# EUROPEAN PATENT APPLICATION

(11) **EP 1 733 733 A1**
(43) Date of publication of application: **20.12.2006**
(21) Application number: 05720016.4
(22) Date of filing: 04.03.2005
(51) Int. Cl.: A61K 38/17, A61K 48/00, A61K 38/28, A61K 31/4353, A61P 3/04, A61P 43/00, A61P 3/10, A61P 9/10

(54) **REGULATOR FOR ADIPONECTIN RECEPTOR EXPRESSION**

(30) Priority: 04.03.2004 US 549561 P
(71) Applicant: Kadowaki, Takashi, Tokyo 113-0034 (JP); Yamauchi, Toshimasa, Tokyo 113-0024 (JP)
(72) Inventor: Kadowaki, Takashi, Tokyo 113-0034 (JP); Yamauchi, Toshimasa, Tokyo 113-0024 (JP)
(74) Representative: Denison, Christopher Marcus
(86) International application number: PCT/JP2005/003744
(87) International publication number: WO 2005/084697

(57) **Abstract**

The present inventors revealed by using fasted and refed mice, that AdipoR1/R2 is a regulator of metabolic sensitivity to nutritional conditions and insulin. They showed that mRNA level ofAdipoR1/R2 increased by STZ treatment, and that this increase was restored by insulin. The present inventors confirmed *in vitro* that insulin reduces AdipoR1/R2 mRNAs in myocytes and such. It was also confirmed that in insulin-resistant models, the AdipoR1/R2 expression was downregulated, and that AMP kinase activation by adiponectin was decreased. The present inventors discovered by using insulin signaling pathway inhibitors, that the downregulation of adiponectin receptors by insulin was mediated by the PI3-kinase/Foxo1 pathway.

SEQUENCE LISTING

<110>  KADOWAKI, TAKASHI
       YAMAUCHI, TOSHIMASA

<120>  THE REGULATORS OF EXPRESSION OF ADIPONECTIN RECEPTORS

<130>  SEN-A0401P

<150>  US 60/549,561
<151>  2004-03-04

<160>  15

<170>  PatentIn version 3.3

<210>  1
<211>  652
<212>  PRT
<213>  Mus musculus

<400>  1

Met Ala Glu Ala Pro Gln Val Val Glu Thr Asp Pro Asp Phe Glu Pro
1               5                   10                  15

Leu Pro Arg Gln Arg Ser Cys Thr Trp Pro Leu Pro Arg Pro Glu Phe
            20                  25                  30

Asn Gln Ser Asn Ser Thr Thr Ser Ser Pro Ala Pro Ser Gly Gly Ala
        35                  40                  45

Ala Ala Asn Pro Asp Ala Ala Ala Ser Leu Ala Ser Ala Ser Ala Val
    50                  55                  60

Ser Thr Asp Phe Met Ser Asn Leu Ser Leu Leu Glu Glu Ser Glu Asp
65                  70                  75                  80

Phe Ala Arg Ala Pro Gly Cys Val Ala Val Ala Ala Ala Ala Ala Ala
                85                  90                  95

Ser Arg Gly Leu Cys Gly Asp Phe Gln Gly Pro Glu Ala Gly Cys Val
            100                 105                 110

His Pro Ala Pro Pro Gln Pro Pro Pro Thr Gly Pro Leu Ser Gln Pro
        115                 120                 125

Pro Pro Val Pro Pro Ser Ala Ala Ala Ala Ala Gly Pro Leu Ala Gly
    130                 135                 140

Gln Pro Arg Lys Thr Ser Ser Ser Arg Arg Asn Ala Trp Gly Asn Leu
145                 150                 155                 160

Ser Tyr Ala Asp Leu Ile Thr Lys Ala Ile Glu Ser Ser Ala Glu Lys
                165                 170                 175

Arg Leu Thr Leu Ser Gln Ile Tyr Glu Trp Met Val Lys Ser Val Pro
            180                 185                 190

Tyr Phe Lys Asp Lys Gly Asp Ser Asn Ser Ser Ala Gly Trp Lys Asn
        195                 200                 205

Ser Ile Arg His Asn Leu Ser Leu His Ser Lys Phe Ile Arg Val Gln
    210                 215                 220

Asn Glu Gly Thr Gly Lys Ser Ser Trp Trp Met Leu Asn Pro Glu Gly
225                 230                 235                 240

Gly Lys Ser Gly Lys Ser Pro Arg Arg Arg Ala Ala Ser Met Asp Asn
                245                 250                 255

Asn Ser Lys Phe Ala Lys Ser Arg Gly Arg Ala Ala Lys Lys Lys Ala
            260                 265                 270

Ser Leu Gln Ser Gly Gln Glu Gly Pro Gly Asp Ser Pro Gly Ser Gln
        275                 280                 285

Phe Ser Lys Trp Pro Ala Ser Pro Gly Ser His Ser Asn Asp Asp Phe
    290                 295                 300

Asp Asn Trp Ser Thr Phe Arg Pro Arg Thr Ser Ser Asn Ala Ser Thr
305                 310                 315                 320

Ile Ser Gly Arg Leu Ser Pro Ile Met Thr Glu Gln Asp Asp Leu Gly
                325                 330                 335

Asp Gly Asp Val His Ser Leu Val Tyr Pro Pro Ser Ala Ala Lys Met
            340                 345                 350

Ala Ser Thr Leu Pro Ser Leu Ser Glu Ile Ser Asn Pro Glu Asn Met
        355                 360                 365

Glu Asn Leu Leu Asp Asn Leu Asn Leu Leu Ser Ser Pro Thr Ser Leu
    370                 375                 380

Thr Val Ser Thr Gln Ser Ser Pro Gly Ser Met Met Gln Gln Thr Pro
385                 390                 395                 400

Cys Tyr Ser Phe Ala Pro Pro Asn Thr Ser Leu Asn Ser Pro Ser Pro
                405                 410                 415

Asn Tyr Ser Lys Tyr Thr Tyr Gly Gln Ser Ser Met Ser Pro Leu Pro
            420                 425                 430

Gln Met Pro Met Gln Thr Leu Gln Asp Ser Lys Ser Ser Tyr Gly Gly
        435                 440                 445

Leu Asn Gln Tyr Asn Cys Ala Pro Gly Leu Leu Lys Glu Leu Leu Thr
    450                 455                 460

Ser Asp Ser Pro Pro His Asn Asp Ile Met Ser Pro Val Asp Pro Gly
465                 470                 475                 480

Val Ala Gln Pro Asn Ser Arg Val Leu Gly Gln Asn Val Met Met Gly
                485                 490                 495

Pro Asn Ser Val Met Pro Ala Tyr Gly Ser Gln Ala Ser His Asn Lys
            500                 505                 510

Met Met Asn Pro Ser Ser His Thr His Pro Gly His Ala Gln Gln Thr
        515                 520                 525

Ala Ser Val Asn Gly Arg Thr Leu Pro His Val Val Asn Thr Met Pro
    530                 535                 540

His Thr Ser Ala Met Asn Arg Leu Thr Pro Val Lys Thr Pro Leu Gln
545                 550                 555                 560

Val Pro Leu Ser His Pro Met Gln Met Ser Ala Leu Gly Ser Tyr Ser
                565                 570                 575

Ser Val Ser Ser Cys Asn Gly Tyr Gly Arg Met Gly Val Leu His Gln
            580                 585                 590

Glu Lys Leu Pro Ser Asp Leu Asp Gly Met Phe Ile Glu Arg Leu Asp
        595                 600                 605

Cys Asp Met Glu Ser Ile Ile Arg Asn Asp Pro Met Asp Gly Asp Thr
    610                 615                 620

Leu Asp Phe Asn Phe Asp Asn Val Leu Pro Asn Gln Ser Phe Pro His
625                 630                 635                 640

Ser Val Lys Thr Thr Thr His Ser Trp Val Ser Gly
                645                 650

<210>  2
<211>  2103
<212>  DNA
<213>  Mus musculus

<220>
<221>  CDS
<222>  (7)..(1965)

<400>  2
gtcacc atg gcc gag gcg ccc cag gtg gtg gag acc gac ccg gac ttc        48
       Met Ala Glu Ala Pro Gln Val Val Glu Thr Asp Pro Asp Phe
       1               5                   10

gag ccg ctg ccc cgg cag cgc tcc tgt acc tgg ccg ctg ccc agg ccg        96
Glu Pro Leu Pro Arg Gln Arg Ser Cys Thr Trp Pro Leu Pro Arg Pro
15                  20                  25                  30

gag ttt aac cag tcc aac tcg acc acc tcc agt ccg gcg ccg tcg ggc       144
Glu Phe Asn Gln Ser Asn Ser Thr Thr Ser Ser Pro Ala Pro Ser Gly
                35                  40                  45

ggc gcg gcc gcc aac ccc gac gcc gcg gcg agc ctg gcc tcg gcg tcc       192
Gly Ala Ala Ala Asn Pro Asp Ala Ala Ala Ser Leu Ala Ser Ala Ser
            50                  55                  60

gct gtc agc acc gac ttt atg agc aac ctg agc ctg ctg gag gag agt       240
Ala Val Ser Thr Asp Phe Met Ser Asn Leu Ser Leu Leu Glu Glu Ser
        65                  70                  75

gag gac ttc gcg cgg gcg cca ggc tgc gtg gcc gtg gcg gcg gcg gct       288
Glu Asp Phe Ala Arg Ala Pro Gly Cys Val Ala Val Ala Ala Ala Ala

80                  85                  90

gcg gcc agc agg ggc ctg tgc ggg gac ttc cag ggc ccc gag gcg ggc          336
Ala Ala Ser Arg Gly Leu Cys Gly Asp Phe Gln Gly Pro Glu Ala Gly
95                  100                 105                 110

tgc gtg cac cca gcg ccg cca cag ccc cca ccg acc ggg ccg ctg tcg          384
Cys Val His Pro Ala Pro Pro Gln Pro Pro Pro Thr Gly Pro Leu Ser
                115                 120                 125

cag ccc cca ccc gtg cct ccc tcc gct gcc gcc gcc gcg ggg cca ctc          432
Gln Pro Pro Pro Val Pro Pro Ser Ala Ala Ala Ala Ala Gly Pro Leu
            130                 135                 140

gcg gga cag ccg cgc aag acc agc tcg tcg cgc cgc aac gcg tgg ggc          480
Ala Gly Gln Pro Arg Lys Thr Ser Ser Ser Arg Arg Asn Ala Trp Gly
        145                 150                 155

aac ctg tcg tac gcc gac ctc atc acc aag gcc atc gag agc tca gcc          528
Asn Leu Ser Tyr Ala Asp Leu Ile Thr Lys Ala Ile Glu Ser Ser Ala
    160                 165                 170

gag aag agg ctc acc ctg tcg cag atc tac gag tgg atg gtg aag agc          576
Glu Lys Arg Leu Thr Leu Ser Gln Ile Tyr Glu Trp Met Val Lys Ser
175                 180                 185                 190

gtg ccc tac ttc aag gat aag ggc gac agc aac agc tcg gcg ggc tgg          624
Val Pro Tyr Phe Lys Asp Lys Gly Asp Ser Asn Ser Ser Ala Gly Trp
                195                 200                 205

aag aat tca att cgc cac aat ctg tcc ctt cac agc aag ttt att cga          672
Lys Asn Ser Ile Arg His Asn Leu Ser Leu His Ser Lys Phe Ile Arg
            210                 215                 220

gtg cag aat gaa gga act gga aag agt tct tgg tgg atg ctc aat cca          720
Val Gln Asn Glu Gly Thr Gly Lys Ser Ser Trp Trp Met Leu Asn Pro
        225                 230                 235

gag gga ggc aag agc gga aaa tca ccc cgg aga aga gct gcg tcc atg          768
Glu Gly Gly Lys Ser Gly Lys Ser Pro Arg Arg Arg Ala Ala Ser Met
    240                 245                 250

gac aac aac agt aaa ttt gct aag agc cga ggg cgg gct gct aag aaa          816
Asp Asn Asn Ser Lys Phe Ala Lys Ser Arg Gly Arg Ala Ala Lys Lys
255                 260                 265                 270

aaa gca tct ctc cag tct ggg caa gag ggt cct gga gac agc cct ggg          864
Lys Ala Ser Leu Gln Ser Gly Gln Glu Gly Pro Gly Asp Ser Pro Gly
                275                 280                 285

tct cag ttt tct aag tgg cct gcg agt cct ggg tcc cac agc aac gat          912
Ser Gln Phe Ser Lys Trp Pro Ala Ser Pro Gly Ser His Ser Asn Asp
            290                 295                 300

gac ttt gat aac tgg agt aca ttt cgt cct cga acc agc tca aat gct          960

Asp Phe Asp Asn Trp Ser Thr Phe Arg Pro Arg Thr Ser Ser Asn Ala
        305                 310                 315

agt acc atc agt ggg aga ctt tct ccc atc atg aca gag cag gat gac        1008
Ser Thr Ile Ser Gly Arg Leu Ser Pro Ile Met Thr Glu Gln Asp Asp
    320                 325                 330

ctg gga gat ggg gac gtg cat tcc ctg gtg tat cca ccc tct gct gcc        1056
Leu Gly Asp Gly Asp Val His Ser Leu Val Tyr Pro Pro Ser Ala Ala
335                 340                 345                 350

aag atg gcg tct acg ctg ccc agt ctg tct gaa atc agc aat cca gaa        1104
Lys Met Ala Ser Thr Leu Pro Ser Leu Ser Glu Ile Ser Asn Pro Glu
                355                 360                 365

aac atg gag aac ctt ctg gat aat ctc aac ctt ctc tcg tcc cca aca        1152
Asn Met Glu Asn Leu Leu Asp Asn Leu Asn Leu Leu Ser Ser Pro Thr
            370                 375                 380

tct tta act gtg tcc acc cag tcc tcg cct ggc agc atg atg cag cag        1200
Ser Leu Thr Val Ser Thr Gln Ser Ser Pro Gly Ser Met Met Gln Gln
        385                 390                 395

aca cca tgc tat tcg ttt gca ccg cca aac acc agt cta aat tca ccc        1248
Thr Pro Cys Tyr Ser Phe Ala Pro Pro Asn Thr Ser Leu Asn Ser Pro
    400                 405                 410

agt cca aac tac tca aag tac aca tac ggc caa tcc agc atg agc cct        1296
Ser Pro Asn Tyr Ser Lys Tyr Thr Tyr Gly Gln Ser Ser Met Ser Pro
415                 420                 425                 430

ttg ccc cag atg cct atg cag aca ctt cag gac agc aaa tca agt tac        1344
Leu Pro Gln Met Pro Met Gln Thr Leu Gln Asp Ser Lys Ser Ser Tyr
                435                 440                 445

gga gga ttg aac cag tat aac tgt gcc cca gga ctc ttg aaa gag ttg        1392
Gly Gly Leu Asn Gln Tyr Asn Cys Ala Pro Gly Leu Leu Lys Glu Leu
            450                 455                 460

ttg act tct gac tct cct ccc cac aat gac att atg tca ccg gtt gat        1440
Leu Thr Ser Asp Ser Pro Pro His Asn Asp Ile Met Ser Pro Val Asp
        465                 470                 475

ccc gga gtg gcc caa ccc aac agt cgg gtc ctg ggc caa aat gta atg        1488
Pro Gly Val Ala Gln Pro Asn Ser Arg Val Leu Gly Gln Asn Val Met
    480                 485                 490

atg ggc cct aat tcg gtc atg cca gcg tat ggc agc cag gca tct cat        1536
Met Gly Pro Asn Ser Val Met Pro Ala Tyr Gly Ser Gln Ala Ser His
495                 500                 505                 510

aac aaa atg atg aac ccc agc tcc cac acc cac cct gga cat gca cag        1584
Asn Lys Met Met Asn Pro Ser Ser His Thr His Pro Gly His Ala Gln
                515                 520                 525

caa acg gct tcg gtc aac ggc cgt acc ctg ccc cat gtg gtg aac acc     1632
Gln Thr Ala Ser Val Asn Gly Arg Thr Leu Pro His Val Val Asn Thr
            530                 535                 540

atg cct cac aca tct gcc atg aac cgc ttg acc ccc gtg aag aca cct     1680
Met Pro His Thr Ser Ala Met Asn Arg Leu Thr Pro Val Lys Thr Pro
        545                 550                 555

tta caa gtg cct ctg tcc cac ccc atg cag atg agt gcc ctg ggc agc     1728
Leu Gln Val Pro Leu Ser His Pro Met Gln Met Ser Ala Leu Gly Ser
    560                 565                 570

tac tcc tcg gtg agc agc tgc aat ggc tat ggt agg atg ggt gtc ctc     1776
Tyr Ser Ser Val Ser Ser Cys Asn Gly Tyr Gly Arg Met Gly Val Leu
575                 580                 585                 590

cac cag gag aag ctc cca agt gac ttg gat ggc atg ttt att gag cgc     1824
His Gln Glu Lys Leu Pro Ser Asp Leu Asp Gly Met Phe Ile Glu Arg
                595                 600                 605

ttg gac tgt gac atg gag tcc atc att cgg aat gac ccc atg gat gga     1872
Leu Asp Cys Asp Met Glu Ser Ile Ile Arg Asn Asp Pro Met Asp Gly
            610                 615                 620

gat acc ttg gat ttt aac ttt gat aat gtg ttg ccc aac caa agc ttc     1920
Asp Thr Leu Asp Phe Asn Phe Asp Asn Val Leu Pro Asn Gln Ser Phe
        625                 630                 635

cca cac agt gtc aag act aca aca cac agc tgg gtg tca ggc taa         1965
Pro His Ser Val Lys Thr Thr Thr His Ser Trp Val Ser Gly
    640                 645                 650

gagtttagtg agcaggctac atttaaaagt ccttcagatt gtctgacagc aggaactgag   2025

gagcagtcca aagatgccct tcacccctcc ttatagtttt caagattaaa aaaaaaaaaa   2085

aaaaaaaaaa aaaaaaaa                                                 2103

<210>  3
<211>  652
<212>  PRT
<213>  Mus musculus

<400>  3

Met Ala Glu Ala Pro Gln Val Val Glu Thr Asp Pro Asp Phe Glu Pro
1               5                   10                  15

Leu Pro Arg Gln Arg Ser Cys Ala Trp Pro Leu Pro Arg Pro Glu Phe
            20                  25                  30

Asn Gln Ser Asn Ser Thr Thr Ser Ser Pro Ala Pro Ser Gly Gly Ala
        35                  40                  45

Ala Ala Asn Pro Asp Ala Ala Ala Ser Leu Ala Ser Ala Ser Ala Val
    50                  55                  60

Ser Thr Asp Phe Met Ser Asn Leu Ser Leu Leu Glu Glu Ser Glu Asp
65                  70                  75                  80

Phe Ala Arg Ala Pro Gly Cys Val Ala Val Ala Ala Ala Ala Ala Ala
                85                  90                  95

Ser Arg Gly Leu Cys Gly Asp Phe Gln Gly Pro Glu Ala Gly Cys Val
            100                 105                 110

His Pro Ala Pro Pro Gln Pro Pro Pro Thr Gly Pro Leu Ser Gln Pro
        115                 120                 125

Pro Pro Val Pro Pro Ser Ala Ala Ala Ala Ala Gly Pro Leu Ala Gly
    130                 135                 140

Gln Pro Arg Lys Thr Ser Ser Ser Arg Arg Asn Ala Trp Gly Asn Leu
145                 150                 155                 160

Ser Tyr Ala Asp Leu Ile Thr Lys Ala Ile Glu Ser Ser Ala Glu Lys
                165                 170                 175

Arg Leu Thr Leu Ser Gln Ile Tyr Glu Trp Met Val Lys Ser Val Pro
            180                 185                 190

Tyr Phe Lys Asp Lys Gly Asp Ser Asn Ser Ser Ala Gly Trp Lys Asn
        195                 200                 205

Ser Ile Arg His Asn Leu Ser Leu His Ser Lys Phe Ile Arg Val Gln
    210                 215                 220

Asn Glu Gly Thr Gly Lys Ser Ser Trp Trp Met Leu Asn Pro Glu Gly
225                 230                 235                 240

Gly Lys Ser Gly Lys Ser Pro Arg Arg Arg Ala Ala Asp Met Asp Asn
                245                 250                 255

Asn Ser Lys Phe Ala Lys Ser Arg Gly Arg Ala Ala Lys Lys Lys Ala
            260                 265                 270

Ser Leu Gln Ser Gly Gln Glu Gly Pro Gly Asp Ser Pro Gly Ser Gln
        275                 280                 285

Phe Ser Lys Trp Pro Ala Ser Pro Gly Ser His Ser Asn Asp Asp Phe
    290                 295                 300

Asp Asn Trp Ser Thr Phe Arg Pro Arg Thr Ser Ala Asn Ala Ser Thr
305                 310                 315                 320

Ile Ser Gly Arg Leu Ser Pro Ile Met Thr Glu Gln Asp Asp Leu Gly
                325                 330                 335

Asp Gly Asp Val His Ser Leu Val Tyr Pro Pro Ser Ala Ala Lys Met
            340                 345                 350

Ala Ser Thr Leu Pro Ser Leu Ser Glu Ile Ser Asn Pro Glu Asn Met
        355                 360                 365

Glu Asn Leu Leu Asp Asn Leu Asn Leu Leu Ser Ser Pro Thr Ser Leu
    370                 375                 380

Thr Val Ser Thr Gln Ser Ser Pro Gly Ser Met Met Gln Gln Thr Pro
385                 390                 395                 400

Cys Tyr Ser Phe Ala Pro Pro Asn Thr Ser Leu Asn Ser Pro Ser Pro
                405                 410                 415

Asn Tyr Ser Lys Tyr Thr Tyr Gly Gln Ser Ser Met Ser Pro Leu Pro
            420                 425                 430

Gln Met Pro Met Gln Thr Leu Gln Asp Ser Lys Ser Ser Tyr Gly Gly
        435                 440                 445

Leu Asn Gln Tyr Asn Cys Ala Pro Gly Leu Leu Lys Glu Leu Leu Thr
    450                 455                 460

Ser Asp Ser Pro Pro His Asn Asp Ile Met Ser Pro Val Asp Pro Gly
465                 470                 475                 480

Val Ala Gln Pro Asn Ser Arg Val Leu Gly Gln Asn Val Met Met Gly
                485                 490                 495

Pro Asn Ser Val Met Pro Ala Tyr Gly Ser Gln Ala Ser His Asn Lys
            500                 505                 510

Met Met Asn Pro Ser Ser His Thr His Pro Gly His Ala Gln Gln Thr
        515                 520                 525

Ala Ser Val Asn Gly Arg Thr Leu Pro His Val Val Asn Thr Met Pro
    530                 535                 540

His Thr Ser Ala Met Asn Arg Leu Thr Pro Val Lys Thr Pro Leu Gln
545                 550                 555                 560

Val Pro Leu Ser His Pro Met Gln Met Ser Ala Leu Gly Ser Tyr Ser
                565                 570                 575

Ser Val Ser Ser Cys Asn Gly Tyr Gly Arg Met Gly Val Leu His Gln
            580                 585                 590

Glu Lys Leu Pro Ser Asp Leu Asp Gly Met Phe Ile Glu Arg Leu Asp
        595                 600                 605

Cys Asp Met Glu Ser Ile Ile Arg Asn Asp Pro Met Asp Gly Asp Thr
    610                 615                 620

Leu Asp Phe Asn Phe Asp Asn Val Leu Pro Asn Gln Ser Phe Pro His
625                 630                 635                 640

Ser Val Lys Thr Thr Thr His Ser Trp Val Ser Gly
                645                 650

<210> 4
<211> 655
<212> PRT
<213> Homo sapiens

<400> 4

Met Ala Glu Ala Pro Gln Val Val Glu Ile Asp Pro Asp Phe Glu Pro
1               5                   10                  15

Leu Pro Arg Pro Arg Ser Cys Thr Trp Pro Leu Pro Arg Pro Glu Phe
            20                  25                  30

Ser Gln Ser Asn Ser Ala Thr Ser Ser Pro Ala Pro Ser Gly Ser Ala
        35                  40                  45

Ala Ala Asn Pro Asp Ala Ala Ala Gly Leu Pro Ser Ala Ser Ala Ala
    50                  55                  60

Ala Val Ser Ala Asp Phe Met Ser Asn Leu Ser Leu Leu Glu Glu Ser
65                  70                  75                  80

Glu Asp Phe Pro Gln Ala Pro Gly Ser Val Ala Ala Ala Val Ala Ala
                85                  90                  95

Ala Ala Ala Ala Ala Ala Thr Gly Gly Leu Cys Gly Asp Phe Gln Gly
            100                 105                 110

Pro Glu Ala Gly Cys Leu His Pro Ala Pro Pro Gln Pro Pro Pro Pro
        115                 120                 125

Gly Pro Leu Ser Gln His Pro Pro Val Pro Pro Ala Ala Ala Gly Pro
    130                 135                 140

Leu Ala Gly Gln Pro Arg Lys Ser Ser Ser Ser Arg Arg Asn Ala Trp
145                 150                 155                 160

Gly Asn Leu Ser Tyr Ala Asp Leu Ile Thr Lys Ala Ile Glu Ser Ser
                165                 170                 175

Ala Glu Lys Arg Leu Thr Leu Ser Gln Ile Tyr Glu Trp Met Val Lys
            180                 185                 190

Ser Val Pro Tyr Phe Lys Asp Lys Gly Asp Ser Asn Ser Ser Ala Gly
        195                 200                 205

Trp Lys Asn Ser Ile Arg His Asn Leu Ser Leu His Ser Lys Phe Ile
    210                 215                 220

Arg Val Gln Asn Glu Gly Thr Gly Lys Ser Ser Trp Trp Met Leu Asn

225                 230                 235                 240

Pro Glu Gly Gly Lys Ser Gly Lys Ser Pro Arg Arg Arg Ala Ala Ser
                245                 250                 255

Met Asp Asn Asn Ser Lys Phe Ala Lys Ser Arg Ser Arg Ala Ala Lys
            260                 265                 270

Lys Lys Ala Ser Leu Gln Ser Gly Gln Glu Gly Ala Gly Asp Ser Pro
        275                 280                 285

Gly Ser Gln Phe Ser Lys Trp Pro Ala Ser Pro Gly Ser His Ser Asn
    290                 295                 300

Asp Asp Phe Asp Asn Trp Ser Thr Phe Arg Pro Arg Thr Ser Ser Asn
305                 310                 315                 320

Ala Ser Thr Ile Ser Gly Arg Leu Ser Pro Ile Met Thr Glu Gln Asp
                325                 330                 335

Asp Leu Gly Glu Gly Asp Val His Ser Met Val Tyr Pro Pro Ser Ala
            340                 345                 350

Ala Lys Met Ala Ser Thr Leu Pro Ser Leu Ser Glu Ile Ser Asn Pro
        355                 360                 365

Glu Asn Met Glu Asn Leu Leu Asp Asn Leu Asn Leu Leu Ser Ser Pro
    370                 375                 380

Thr Ser Leu Thr Val Ser Thr Gln Ser Ser Pro Gly Thr Met Met Gln
385                 390                 395                 400

Gln Thr Pro Cys Tyr Ser Phe Ala Pro Pro Asn Thr Ser Leu Asn Ser
                405                 410                 415

Pro Ser Pro Asn Tyr Gln Lys Tyr Thr Tyr Gly Gln Ser Ser Met Ser
            420                 425                 430

Pro Leu Pro Gln Met Pro Ile Gln Thr Leu Gln Asp Asn Lys Ser Ser
        435                 440                 445

Tyr Gly Gly Met Ser Gln Tyr Asn Cys Ala Pro Gly Leu Leu Lys Glu
    450                 455                 460

Leu Leu Thr Ser Asp Ser Pro Pro His Asn Asp Ile Met Thr Pro Val
465                 470                 475                 480

Asp Pro Gly Val Ala Gln Pro Asn Ser Arg Val Leu Gly Gln Asn Val
                485                 490                 495

Met Met Gly Pro Asn Ser Val Met Ser Thr Tyr Gly Ser Gln Ala Ser
            500                 505                 510

His Asn Lys Met Met Asn Pro Ser Ser His Thr His Pro Gly His Ala
        515                 520                 525

Gln Gln Thr Ser Ala Val Asn Gly Arg Pro Leu Pro His Thr Val Ser
    530                 535                 540

Thr Met Pro His Thr Ser Gly Met Asn Arg Leu Thr Gln Val Lys Thr
545                 550                 555                 560

Pro Val Gln Val Pro Leu Pro His Pro Met Gln Met Ser Ala Leu Gly
                565                 570                 575

Gly Tyr Ser Ser Val Ser Ser Cys Asn Gly Tyr Gly Arg Met Gly Leu
            580                 585                 590

Leu His Gln Glu Lys Leu Pro Ser Asp Leu Asp Gly Met Phe Ile Glu
        595                 600                 605

Arg Leu Asp Cys Asp Met Glu Ser Ile Ile Arg Asn Asp Leu Met Asp
    610                 615                 620

Gly Asp Thr Leu Asp Phe Asn Phe Asp Asn Val Leu Pro Asn Gln Ser
625                 630                 635                 640

Phe Pro His Ser Val Lys Thr Thr Thr His Ser Trp Val Ser Gly
                645                 650                 655

<210>  5
<211>  5723
<212>  DNA

<213>  Homo sapiens

<220>
<221>  CDS
<222>  (386)..(2353)

<400>  5
gcagccgcca cattcaacag gcagcagcgc agcgggcgcg ccgctgggga gagcaagcgg      60

cccgcggcgt ccgtccgtcc ttccgtccgc ggccctgtca gctggagcgc ggcgcaggct     120

ctgccccggc ccggcggctc tggccggccg tccagtccgt gcggcggacc ccgaggagcc     180

tcgatgtgga tggccccgcg aagttaagtt ctgggctcgc gcttccactc cgccgcgcct     240

tcctcccagt ttccgtccgc tcgccgcacc ggcttcgttc ccccaaatct cggaccgtcc     300

cttcgcgccc cctccccgtc cgcccccagt gctgcgttct ccccctcttg gctctcctgc     360

ggctggggga ggggcggggg tcacc atg gcc gag gcg cct cag gtg gtg gag      412
                            Met Ala Glu Ala Pro Gln Val Val Glu
                            1               5

atc gac ccg gac ttc gag ccg ctg ccc cgg ccg cgc tcg tgc acc tgg      460
Ile Asp Pro Asp Phe Glu Pro Leu Pro Arg Pro Arg Ser Cys Thr Trp
10                  15                  20                  25

ccg ctg ccc agg ccg gag ttt agc cag tcc aac tcg gcc acc tcc agc      508
Pro Leu Pro Arg Pro Glu Phe Ser Gln Ser Asn Ser Ala Thr Ser Ser
                30                  35                  40

ccg gcg ccg tcg ggc agc gcg gct gcc aac ccc gac gcc gcg gcg ggc      556
Pro Ala Pro Ser Gly Ser Ala Ala Ala Asn Pro Asp Ala Ala Ala Gly
            45                  50                  55

ctg ccc tcg gcc tcg gct gcc gct gtc agc gcc gac ttc atg agc aac      604
Leu Pro Ser Ala Ser Ala Ala Ala Val Ser Ala Asp Phe Met Ser Asn
        60                  65                  70

ctg agc ttg ctg gag gag agc gag gac ttc ccg cag gcg ccc ggc tcc      652
Leu Ser Leu Leu Glu Glu Ser Glu Asp Phe Pro Gln Ala Pro Gly Ser
    75                  80                  85

gtg gcg gcg gcg gtg gcg gcg gcg gcc gcc gcg gcc gcc acc ggg ggg      700
Val Ala Ala Ala Val Ala Ala Ala Ala Ala Ala Ala Ala Thr Gly Gly
90                  95                  100                 105

ctg tgc ggg gac ttc cag ggc ccg gag gcg ggc tgc ctg cac cca gcg      748
Leu Cys Gly Asp Phe Gln Gly Pro Glu Ala Gly Cys Leu His Pro Ala
                110                 115                 120

cca ccg cag ccc ccg ccg ccc ggg ccg ctg tcg cag cac ccg ccg gtg      796
Pro Pro Gln Pro Pro Pro Pro Gly Pro Leu Ser Gln His Pro Pro Val
            125                 130                 135

ccc ccc gcc gcc gct ggg ccg ctc gcg ggg cag ccg cgc aag agc agc 844
Pro Pro Ala Ala Ala Gly Pro Leu Ala Gly Gln Pro Arg Lys Ser Ser
        140                 145                 150

tcg tcc cgc cgc aac gcg tgg ggc aac ctg tcc tac gcc gac ctc atc 892
Ser Ser Arg Arg Asn Ala Trp Gly Asn Leu Ser Tyr Ala Asp Leu Ile
    155                 160                 165

acc aag gcc atc gag agc tcg gcg gag aag cgg ctc acg ctg tcg cag 940
Thr Lys Ala Ile Glu Ser Ser Ala Glu Lys Arg Leu Thr Leu Ser Gln
170                 175                 180                 185

atc tac gag tgg atg gtc aag agc gtg ccc tac ttc aag gat aag ggt 988
Ile Tyr Glu Trp Met Val Lys Ser Val Pro Tyr Phe Lys Asp Lys Gly
                190                 195                 200

gac agc aac agc tcg gcg ggc tgg aag aat tca att cgt cat aat ctg 1036
Asp Ser Asn Ser Ser Ala Gly Trp Lys Asn Ser Ile Arg His Asn Leu
            205                 210                 215

tcc cta cac agc aag ttc att cgt gtg cag aat gaa gga act gga aaa 1084
Ser Leu His Ser Lys Phe Ile Arg Val Gln Asn Glu Gly Thr Gly Lys
        220                 225                 230

agt tct tgg tgg atg ctc aat cca gag ggt ggc aag agc ggg aaa tct 1132
Ser Ser Trp Trp Met Leu Asn Pro Glu Gly Gly Lys Ser Gly Lys Ser
    235                 240                 245

cct agg aga aga gct gca tcc atg gac aac aac agt aaa ttt gct aag 1180
Pro Arg Arg Arg Ala Ala Ser Met Asp Asn Asn Ser Lys Phe Ala Lys
250                 255                 260                 265

agc cga agc cga gct gcc aag aag aaa gca tct ctc cag tct ggc cag 1228
Ser Arg Ser Arg Ala Ala Lys Lys Lys Ala Ser Leu Gln Ser Gly Gln
                270                 275                 280

gag ggt gct ggg gac agc cct gga tca cag ttt tcc aaa tgg cct gca 1276
Glu Gly Ala Gly Asp Ser Pro Gly Ser Gln Phe Ser Lys Trp Pro Ala
            285                 290                 295

agc cct ggc tct cac agc aat gat gac ttt gat aac tgg agt aca ttt 1324
Ser Pro Gly Ser His Ser Asn Asp Asp Phe Asp Asn Trp Ser Thr Phe
        300                 305                 310

cgc cct cga act agc tca aat gct agt act att agt ggg aga ctc tca 1372
Arg Pro Arg Thr Ser Ser Asn Ala Ser Thr Ile Ser Gly Arg Leu Ser
    315                 320                 325

ccc att atg acc gaa cag gat gat ctt gga gaa ggg gat gtg cat tct 1420
Pro Ile Met Thr Glu Gln Asp Asp Leu Gly Glu Gly Asp Val His Ser
330                 335                 340                 345

atg gtg tac ccg cca tct gcc gca aag atg gcc tct act tta ccc agt 1468
Met Val Tyr Pro Pro Ser Ala Ala Lys Met Ala Ser Thr Leu Pro Ser

                350                 355                 360

ctg tct gag ata agc aat ccc gaa aac atg gaa aat ctt ttg gat aat 1516
Leu Ser Glu Ile Ser Asn Pro Glu Asn Met Glu Asn Leu Leu Asp Asn
            365                 370                 375

ctc aac ctt ctc tca tca cca aca tca tta act gtt tcg acc cag tcc 1564
Leu Asn Leu Leu Ser Ser Pro Thr Ser Leu Thr Val Ser Thr Gln Ser
        380                 385                 390

tca cct ggc acc atg atg cag cag acg ccg tgc tac tcg ttt gcg cca 1612
Ser Pro Gly Thr Met Met Gln Gln Thr Pro Cys Tyr Ser Phe Ala Pro
    395                 400                 405

cca aac acc agt ttg aat tca ccc agc cca aac tac caa aaa tat aca 1660
Pro Asn Thr Ser Leu Asn Ser Pro Ser Pro Asn Tyr Gln Lys Tyr Thr
410                 415                 420                 425

tat ggc caa tcc agc atg agc cct ttg ccc cag atg cct ata caa aca 1708
Tyr Gly Gln Ser Ser Met Ser Pro Leu Pro Gln Met Pro Ile Gln Thr
                430                 435                 440

ctt cag gac aat aag tcg agt tat gga ggt atg agt cag tat aac tgt 1756
Leu Gln Asp Asn Lys Ser Ser Tyr Gly Gly Met Ser Gln Tyr Asn Cys
            445                 450                 455

gcg cct gga ctc ttg aag gag ttg ctg act tct gac tct cct ccc cat 1804
Ala Pro Gly Leu Leu Lys Glu Leu Leu Thr Ser Asp Ser Pro Pro His
        460                 465                 470

aat gac att atg aca cca gtt gat cct ggg gta gcc cag ccc aac agc 1852
Asn Asp Ile Met Thr Pro Val Asp Pro Gly Val Ala Gln Pro Asn Ser
    475                 480                 485

cgg gtt ctg ggc cag aac gtc atg atg ggc cct aat tcg gtc atg tca 1900
Arg Val Leu Gly Gln Asn Val Met Met Gly Pro Asn Ser Val Met Ser
490                 495                 500                 505

acc tat ggc agc cag gca tct cat aac aaa atg atg aat ccc agc tcc 1948
Thr Tyr Gly Ser Gln Ala Ser His Asn Lys Met Met Asn Pro Ser Ser
                510                 515                 520

cat acc cac cct gga cat gct cag cag aca tct gca gtt aac ggg cgt 1996
His Thr His Pro Gly His Ala Gln Gln Thr Ser Ala Val Asn Gly Arg
            525                 530                 535

ccc ctg ccc cac acg gta agc acc atg ccc cac acc tcg ggt atg aac 2044
Pro Leu Pro His Thr Val Ser Thr Met Pro His Thr Ser Gly Met Asn
        540                 545                 550

cgc ctg acc caa gtg aag aca cct gta caa gtg cct ctg ccc cac ccc 2092
Arg Leu Thr Gln Val Lys Thr Pro Val Gln Val Pro Leu Pro His Pro
    555                 560                 565

atg cag atg agt gcc ctg ggg ggc tac tcc tcc gtg agc agc tgc aat 2140

Met Gln Met Ser Ala Leu Gly Gly Tyr Ser Ser Val Ser Ser Cys Asn
570                 575                 580                 585

ggc tat ggc aga atg ggc ctt ctc cac cag gag aag ctc cca agt gac     2188
Gly Tyr Gly Arg Met Gly Leu Leu His Gln Glu Lys Leu Pro Ser Asp
                590                 595                 600

ttg gat ggc atg ttc att gag cgc tta gac tgt gac atg gaa tcc atc     2236
Leu Asp Gly Met Phe Ile Glu Arg Leu Asp Cys Asp Met Glu Ser Ile
            605                 610                 615

att cgg aat gac ctc atg gat gga gat aca ttg gat ttt aac ttt gac     2284
Ile Arg Asn Asp Leu Met Asp Gly Asp Thr Leu Asp Phe Asn Phe Asp
        620                 625                 630

aat gtg ttg ccc aac caa agc ttc cca cac agt gtc aag aca acg aca     2332
Asn Val Leu Pro Asn Gln Ser Phe Pro His Ser Val Lys Thr Thr Thr
    635                 640                 645

cat agc tgg gtg tca ggc tga gggttagtga gcaggttaca cttaaaagta        2383
His Ser Trp Val Ser Gly
650                 655

cttcagattg tctgacagca ggaactgaga gaagcagtcc aaagatgtct ttcaccaact   2443

cccttttagt tttcttggtt aaaaaaaaaa acaaaaaaaa aaaccctcct tttttccttt   2503

tcgtcagact tggcagcaaa gacatttttc ctgtacagga tgtttgccca atgtgtgcag   2563

gttatgtgct gctgtagata aggactgtgc cattggaaat ttcattacaa tgaagtgcca   2623

aactcactac accatataat tgcagaaaag attttcagat cctggtgtgc tttcaagttt   2683

tgtatataag cagtagatac agattgtatt tgtgtgtgtt tttggttttt ctaaatatcc   2743

aattggtcca aggaaagttt atactctttt tgtaatactg tgatgggcct catgtcttga   2803

taagttaaac ttttgtttgt actacctgtt ttctgcggaa ctgacggatc acaaagaact   2863

gaatctccat tctgcatctc cattgaacag ccttggacct gttcacgttg ccacagaatt   2923

cacatgagaa ccaagtagcc tgttatcaat ctgctaaatt aatggacttg ttaaactttt   2983

ggaaaaaaaa agattaaatg ccagctttgt acaggtcttt tctatttttt tttgtttatt   3043

ttgttatttg caaatttgta caaacattta aatggttcta atttccagat aaatgatttt   3103

tgatgttatt gttgggactt aagaacattt ttggaataga tattgaactg taataatgtt   3163

ttcttaaaac tagagtctac tttgttacat agtcagcttg taaattttgt ggaaccacag   3223

gtatttgggg cagcattcat aattttcatt ttgtattcta actggattag tactaatttt   3283

atacatgctt aactggtttg tacactttgg gatgctactt agtgatgttt ctgactaatc   3343

ttaaatcatt gtaattagta cttgcatatt caacgtttca ggccctggtt gggcaggaaa 3403
gtgatgtata gttatggaca ctttgcgttt cttatttagg ataacttaat atgtttttat 3463
gtatgtattt taaagaaatt tcatctgctt ctactgaact atgcgtactg catagcatca 3523
agtcttctct agagacctct gtagtcctgg gaggcctcat aatgtttgta gatcagaaaa 3583
gggagatctg catctaaagc aatggtcctt tgtcaaacga gggattttga tccacttcac 3643
cattttgagt tgagctttag caaaagtttc ccctcataat tctttgctct tgtttcagtc 3703
caggtggagg ttggttttgt agttctgcct tgaggaatta tgtcaacact catacttcat 3763
ctcattctcc cttctgccct gcagattaga ttacttagca cactgtggaa gtttaagtgg 3823
aaggagggaa tttaaaaatg ggacttgagt ggtttgtaga atttgtgttc ataagttcag 3883
atgggtagca aatggaatag aacttactta aaaattgggg agatttattt gaaaaccagc 3943
tgtaagttgt gcattgagat tatgttaaaa gccttggctt aagaatttga aaatttcttt 4003
agcctgtagc aacctaaact gtaattccta tcattatgtt ttattacttt ccaattacct 4063
gtaactgaca gaccaaatta attggctttg tgtcctattt agtccatcag tattttcaag 4123
tcatgtggaa agcccaaagt catcacaatg aagagaacag gtgcacagca ctgttcctct 4183
tgtgttcttg agaaggatct aatttttctg tatatagccc acatcacact tgctttgtct 4243
tgtatgttaa ttgcatcttc attggcttgg tatttcctaa atgtttaaca agaacacaag 4303
tgttcctgat aagatttcct acagtaagcc agctgtattg taagcttccc accgtgatga 4363
tcattttttt gaagattcat tgaacagcca ccactctatc atcctcattt tggggcagtc 4423
caagacatag ctggttttag aaacccaagt tcctctaagc acagcctccc gggtatgtaa 4483
ctgaacttgg tgccaaagta cttgtgtact aatttctatt actacgtact gtcactttcc 4543
tcccgtgcca ttactgcatc ataatacaag gaacctcaga gcccccattt gttcattaaa 4603
gaggcaacta cagccaaaat cactgttaaa atcttactac ttcatggagt agctcttagg 4663
aaaatatatc ttcctcctga gtctgggtaa ttatacctct cccaagcccc cattgtgtgt 4723
tgaaatcctg tcatgaatcc ttggtagctc tctgagaaca gtgaagtcca gggaaaggca 4783
tctggtctgt ctggaaagca aacattatgt ggcctctggt agtttttttc ctgtaagaat 4843
actgactttc tggagtaatg agtatatatc agttattgta catgattgct ttgtgaaatg 4903
tgcaaatgat atcacctatg cagccttgtt tgatttattt tctctggttt gtactgttat 4963
taaaagcata ttgtattata gagctattca gatattttaa atataaagat gtattgtttc 5023

cgtaatatag acgtatggaa tatatttagg taatagatgt attacttgga aagttctgct 5083

ttgacaaact gacaaagtct aaatgagcac atgtatccca gtgagcagta aatcaatgga 5143

acatcccaag aagaggataa ggatgcttaa aatggaaatc attctccaac gatatacaaa 5203

ttggacttgt tcaactgctg gatatatgct accaataacc ccagccccaa cttaaaattc 5263

ttacattcaa gctcctaaga gttcttaatt tataactaat tttaaaagag aagtttcttt 5323

tctggtttta gtttgggaat aatcattcat taaaaaaaat gtattgtggt ttatgcgaac 5383

agaccaacct ggcattacag ttggcctctc cttgaggtgg gcacagcctg gcagtgtggc 5443

caggggtggc catgtaagtc ccatcaggac gtagtcatgc ctcctgcatt tcgctacccg 5503

agtttagtaa cagtgcagat tccacgttct tgttccgata ctctgagaag tgcctgatgt 5563

tgatgtactt acagacacaa gaacaatctt tgctataatt gtataaagcc ataaatgtac 5623

ataaattatg tttaaatggc ttggtgtctt tcttttctaa ttatgcagaa taagctcttt 5683

attaggaatt ttttgtgaag ctattaaata cttgagttaa 5723

<210> 6
<211> 110
<212> PRT
<213> Mus musculus

<400> 6

Met Ala Leu Trp Met Arg Phe Leu Pro Leu Leu Ala Leu Leu Phe Leu
1               5                   10                  15

Trp Glu Ser His Pro Thr Gln Ala Phe Val Lys Gln His Leu Cys Gly
            20                  25                  30

Ser His Leu Val Glu Ala Leu Tyr Leu Val Cys Gly Glu Arg Gly Phe
        35                  40                  45

Phe Tyr Thr Pro Met Ser Arg Arg Glu Val Glu Asp Pro Gln Val Ala
    50                  55                  60

Gln Leu Glu Leu Gly Gly Gly Pro Gly Ala Gly Asp Leu Gln Thr Leu
65                  70                  75                  80

Ala Leu Glu Val Ala Gln Gln Lys Arg Gly Ile Val Asp Gln Cys Cys

85                  90                  95

Thr Ser Ile Cys Ser Leu Tyr Gln Leu Glu Asn Tyr Cys Asn
            100                 105                 110

<210>  7
<211>  460
<212>  DNA
<213>  Mus musculus

<220>
<221>  CDS
<222>  (75)..(407)

<400>  7
gagccctaag tgatccgcta caatcaaaaa ccatcagcaa gcaggaagcc tatcttccag      60

gttattgttt caac atg gcc ctg tgg atg cgc ttc ctg ccc ctg ctg gcc      110
                Met Ala Leu Trp Met Arg Phe Leu Pro Leu Leu Ala
                1               5                   10

ctg ctc ttc ctc tgg gag tcc cac ccc acc cag gct ttt gtc aag cag      158
Leu Leu Phe Leu Trp Glu Ser His Pro Thr Gln Ala Phe Val Lys Gln
        15                  20                  25

cac ctt tgt ggt tcc cac ctg gtg gag gct ctc tac ctg gtg tgt ggg      206
His Leu Cys Gly Ser His Leu Val Glu Ala Leu Tyr Leu Val Cys Gly
    30                  35                  40

gag cgt ggc ttc ttc tac aca ccc atg tcc cgc cgt gaa gtg gag gac      254
Glu Arg Gly Phe Phe Tyr Thr Pro Met Ser Arg Arg Glu Val Glu Asp
45                  50                  55                  60

cca caa gtg gca caa ctg gag ctg ggt gga ggc ccg gga gca ggt gac      302
Pro Gln Val Ala Gln Leu Glu Leu Gly Gly Gly Pro Gly Ala Gly Asp
                65                  70                  75

ctt cag acc ttg gca ctg gag gtg gcc cag cag aag cgt ggc att gta      350
Leu Gln Thr Leu Ala Leu Glu Val Ala Gln Gln Lys Arg Gly Ile Val
            80                  85                  90

gat cag tgc tgc acc agc atc tgc tcc ctc tac cag ctg gag aac tac      398
Asp Gln Cys Cys Thr Ser Ile Cys Ser Leu Tyr Gln Leu Glu Asn Tyr
        95                  100                 105

tgc aac tag acccaccact acccagccta cccctctgca atgaataaaa      447
Cys Asn
    110

cctttgaatg agg                                                    460

<210>  8
<211>  110
<212>  PRT
<213>  Homo sapiens

<400>  8

<210>  9
<211>  450
<212>  DNA
<213>  Homo sapiens

<220>
<221>  CDS
<222>  (45)..(377)

<400>  9
gctgcatcag aagaggccat caagcacatc actgtccttc tgcc atg gcc ctg tgg      56
atg cgc ctc ctg ccc ctg ctg gcg ctg ctg gcc ctc tgg gga cct gac      104
cca gcc gca gcc ttt gtg aac caa cac ctg tgc ggc tca cac ctg gtg     152
gaa gct ctc tac cta gtg tgc ggg gaa cga ggc ttc ttc tac aca ccc     200
aag acc cgc cgg gag gca gag gac ctg cag gtg ggg cag gtg gag ctg     248
ggc ggg ggc cct ggt gca ggc agc ctg cag ccc ttg gcc ctg gag ggg     296
tcc ctg cag aag cgt ggc att gtg gaa caa tgc tgt acc agc atc tgc     344
tcc ctc tac cag ctg gag aac tac tgc aac tag acgcagcccg caggcagccc     397
cccacccgcc gcctcctgca ccgagagaga tggaataaag cccttgaacc agc     450


<210>  10
<211>  22
<212>  DNA
<213>  Artificial

<220>
<223>  an artificially synthesized primer sequence

<400>  10
acgttggaga gtcatcccgt at                                            22


<210>  11
<211>  21
<212>  DNA
<213>  Artificial

<220>
<223>  an artificially synthesized primer sequence

<400>  11
ctctgtgtgg atgcggaaga t                                             21


<210>  12
<211>  25
<212>  DNA

<213>  Artificial

<220>
<223>  an artificially synthesized probe sequence

<400>  12
cctgctacat ggccacagac cacct                                          25


<210>  13
<211>  23
<212>  DNA
<213>  Artificial

<220>
<223>  an artificially synthesized primer sequence

<400>  13
tcccaggaag atgaagggtt tat                                            23


<210>  14
<211>  22
<212>  DNA
<213>  Artificial

<220>
<223>  an artificially synthesized primer sequence

<400>  14
ttccattcgt tcgatagcat ga                                             22


<210>  15
<211>  22
<212>  DNA
<213>  Artificial

<220>
<223>  an artificially synthesized probe sequence

<400>  15
atgtccccgc tcctacaggc cc                                             22

## Description

### Technical Field

The present invention relates to therapies for insulin resistance and diabetes that utilize regulation of adiponectin receptor expression.

### Background Art

Adiponectin/Acrp30 (Non-Patent Documents 1-4) is a hormone secreted from adipocytes, which acts as an anti-diabetic (Non-Patent Documents 5-12) and anti-atherogenic (Non-Patent Documents 8, 12, and 13) adipokine. Adiponectin is thought to accentuate insulin-sensitivity by enhancing fatty acid oxidation via the activation of 5'-AMP-activated protein kinase (AMPK) (Non-Patent Documents 10 and 11) and peroxisome proliferator-activated receptor (PPAR)-α (Non-Patent Documents 5, 6, and 12). Recently, the present inventors succeeded in the cloning of cDNAs encoding adiponectin receptors 1 and 2 (AdipoR1, and AdipoR2) (Patent Document 1, Non-Patent Document 14). AdipoR1 is abundantly expressed in skeletal muscle, whereas AdipoR2 is primarily expressed in liver. AdipoR1 and R2 are predicted to contain seven transmembrane domains (Non-Patent Document 14), but are considered to be structurally and functionally distinct from G-protein-coupled receptors (Non-Patent Documents 15-17). AdipoR1 and R2 function as receptors for globular adiponectin and full-length adiponectin, and mediate the enhancement of AMPK activity (Non-Patent Documents 10 and 11), PPARα ligand activity (Non-Patent Document 12), and fatty acid oxidation and glucose uptake by adiponectin (Non-Patent Document 14).

As described above, findings on AdipoR1 and AdipoR2 are starting to accumulate, but it was not known whether the expression of AdipoR1 and R2 changes under physiological and pathophysiological conditions.
[Patent Document 1] International Application Number: PCT/JP2003/007515, International Application Number: WO2004/061108, Patent number: US2004241802 [Non-patent Document 1] Scherer, P.E., Williams, S., Fogliano, M., Baldini, G., and Lodish, H.F. (1995) J. Biol. Chem. 270, 26746-26749
[Non-patent Document 2] Hu, E., Liang, P., and Spiegelman, B.M. (1996) J. Biol. Chem. 271, 10697-10703
[Non-patent Document 3] Maeda, K., Okubo, K., Shimomura, I., Funahashi, T., Matsuzawa, Y., and Matsubara, K. (1996) Biochem. Biophys. Res. Commun. 221, 286-296
[Non-patent Document 4] Nakano, Y., Tobe, T., Choi-Miura, N.H., Mazda, T., and Tomita, M. (1996) J. Biochem. (Tokyo) 120, 802-812
[Non-patent Document 5] Fruebis, J., Tsao, T.S., Javorschi, S., Ebbets-Reed, D., Erickson, M.R., Yen, F.T., Bihain, B.E., and Lodish, H.F. (2001) Proc. Natl. Acad. Sci. USA. 98, 2005-2010
[Non-patent Document 6] Yamauchi, T., Kamon, J., Waki, H., Terauchi, Y, Kubota, N., Hara, K., Mori, Y, Ide, T., Murakami, K., Tsuboyama-Kasaoka, N., Ezaki, O., Akanuma, Y., Gavrilova, O., Vinson, C., Reitman, M.L., Kagechika, H., Shudo, K., Yoda, M., Nakano, Y, Tobe, K., Nagai, R., Kimura, S., Tomita, M., Froguel, P., and Kadowaki, T. (2001) Nat. Med. 7, 941-946
[Non-patent Document 7] Berg, A.H., Combs, T.P., Du, X., Brownlee, M., and Scherer, P.E. (2001) Nat. Med. 7, 947-953
[Non-patent Document 8] Kubota, N., Terauchi, Y., Yamauchi, T., Kubota, T., Moroi, M., Matsui, J., Eto, K., Yamashita, T., Kamon, J., Satoh, H., Yano, W., Froguel, P., Nagai, R., Kimura, S., Kadowaki, T., and Noda, T. (2002) J. Biol. Chem. 277, 25863-25866 [Non-patent Document 9] Maeda, N., Shimomura, I., Kishida, K., Nishizawa, H., Matsuda, M., Nagaretani, H., Furuyama, N., Kondo, H., Takahashi, M., Arita, Y., Komuro, R., Ouchi, N., Kihara, S., Tochino, Y., Okutomi, K., Horie, M., Takeda, S., Aoyama, T., Funahashi, T., and Matsuzawa, Y (2002) Nat. Med. 8,731-737
[Non-patent Document 10] Yamauchi, T., Kamon, J., Minokoshi, Y, Ito, Y, Waki, H., Uchida, S., Yamashita, S., Noda, M., Kita, S., Ueki, K., Eto, K., Akanuma, Y, Froguel, P., Foufelle, F., Ferre, P., Carling, D., Kimura, S., Nagai, R., Kahn, B.B., and Kadowaki, T. (2002) Nat. Med. 8, 1288-1295
[Non-patent Document 11] Tomas, E., Tsao, T.S., Saha, A.K., Murrey, H.E., Zhang, Cc. C., Itani, S.I., Lodish, H.F., and Ruderman, N.B. (2002) Proc. Natl. Acad. Sci. USA. 99, 16309-16313
[Non-patent Document 12] Yamauchi, T., Kamon, J., Waki, H., Imai, Y, Shimozawa, N., Hioki, K., Uchida, S., Ito, Y, Takakuwa, K., Matsui, J., Takata, M., Eto, K., Terauchi, Y., Komeda, K., Tsunoda, M., Murakami, K., Ohnishi, Y., Naitoh, T., Yamamura, K., Ueyama, Y, Froguel, P., Kimura, S., Nagai, R., and Kadowaki, T. (2003) J. Biol. Chem. 278, 2461-2468
[Non-patent Document 13] Ouchi, N., Kihara, S., Arita, Y, Nishida, M., Matsuyama, A., Okamoto, Y, Ishigami, M., Kuriyama, H., Kishida, K., Nishizawa, H., Hotta, K., Muraguchi, M., Ohmoto, Y., Yamashita, S., Funahashi, T., and Matsuzawa, Y. (2001) Circulation 103, 1057-1063
[Non-patent Document 14] Yamauchi, T., Kamon, J., Ito, Y, Tsuchida, A., Yokomizo, T., Kita, S., Sugiyama, T., Miyagishi, M., Hara, K., Tsunoda, M., Murakami, K., Ohteki, T., Uchida, S., Takekawa, S., Waki, H., Tsuno, N.H., Shibata, Y., Terauchi, Y., Froguel, P., Tobe, K., Koyasu, S., Taira, K., Kitamura, T., Shimizu, T., Nagai, R., and Kadowaki, T. (2003) Nature 423, 762-769
[Non-patent Document 15] Wess, J. (1997) FASEB. J. 11, 346-354
[Non-patent Document 16] Yokomizo, T., Izumi, T., Chang, K., Takuwa, Y, and Shimizu, T. (1997) Nature 387, 620-624
[Non-patent Document 17] Scheer, A., Fanelli, F., Costa, T., De Benedetti, P. G., and Cotecchia, S. (1996) EMBO. J. 15, 3566-3578
[Non-patent Document 18] Rakieten, N., Rakieten, MI., and Nadkarni, MV. (1963) Cancer Chemother. Rep. 29, 91-98
[Non-patent Document 19] Yamauchi, T., Waki, H., Kamon, J., Murakami, K., Motojima, K., Komeda, K., Miki, H., Kubota, N., Terauchi, Y, Tsuchida, A., Tsuboyama-Kasaoka, N., Yamauchi, N., Ide, T., Hori, W., Kato, S., Fukayama, M., Akanuma, Y., Ezaki, O., Itai, A., Nagai, R., Kimura, S., Tobe, K., Kagechika, H., Shudo, K., and Kadowaki, T. (2001) J. Clin. Invest. 108, 1001-1013
[Non-patent Document 20] Seglen, PO. (1976) Methods Cell. Biol. 13, 29-83 [Non-patent Document 21] Nakae, J., Park, B.-C., and Accili, D. (1999) J. BIol. Chem. 274, 15982-15985
[Non-patent Document 22] Nakae, J., Kitamura, T., Siliver, DL., and Accili, D. (2001) J. Clin. Invest. 108, 1359-1367
[Non-patent Document 23] Levine, BS., Henry, MC., Port, CD., and Rosen, E. (1980) Drug. Chem. Toxicol. 3, 201-212
[Non-patent Document 24] Herzig, S., Long, F., Jhala, US., Hedrick, S., Quinn, R., Bauer, A., Rudolph, D., Schutz, G., Yoon, C., Puigserver, P., Spiegelman, B., and Montminy, M. Nature (2001) 13, 179-83
[Non-patent Document 25] Friedman, JE., Sun, Y., Ishizuka, T., Farrell, CJ., McCormack, SE., Herron, LM., Hakimi, P., Lechner, P., and Yun, JS. J. Biol. Chem. (1997) 272, 31475-31481
[Non-patent Document 26] Shepherd, P. R., Withers, D. J., and Siddle, K. Biochem. J. (1998) 333, 471-490
[Non-patent Document 27] Kadowaki, T. J. Clin. Invest. (2000) 106, 459-465
[Non-patent Document 28] Virkamaki, A., Ueki, K., and Kahn, CR. J. Clin. Invest. (1999) 103, 931-943
[Non-patent Document 29] Guo, S., Rena, G., Cichy, S., He, X., Cohen, P., and Unterman, T. J. Biol. Chem. (1999) 274, 17184-17192
[Non-patent Document 30] Biggs, W. H., Meisenhelder, J., Hunter, T., Cavenee, W. K., and Arden, K. C. Proc. Natl. Acad. Sci. U.S.A. (1999) 96, 7421-7426
[Non-patent Document 31] Takaishi, H., Konishi, H., Matsuzaki, H., Ono, Y., Shirai, Y., Saito, N., Kitamura, T., Ogawa, W., Kasuga, M., Kikkawa, U., and Nishizuka, Y. Proc. Natl. Acad. Sci. U.S.A. (1999) 96, 11836-11841
[Non-patent Document 32] Brunet, A., Bonni, A., Zigmond, M. J., Lin, M. Z., Juo, P., Hu, L. S., Anderson, M. J., Arden, K. C., Blenis, J., and Greenberg, M. E. Cell (1999) 96, 857-868

### Disclosure of the Invention

The first objective of the present invention is to elucidate the physiological and pathophysiological functions of adiponectin receptors (AdipoR1 and AdipoR2). The second objective is to find a means for applying adiponectin receptors in diabetes treatment, based on the above functions.

In order to solve the above issues, the present inventors examined the expression levels of AdipoR1 and AdipoR2 and the insulin levels in mice under fasting and refeeding conditions. The AdipoR1/R2 expressions dramatically changed by fasting and refeeding. Such changes in AdipoR1/R2 expressions were suggested to be inversely correlated with *in vivo* plasma insulin levels, indicating that AdipoR1/R2 may be important regulators of the metabolic sensitivity to nutritive conditions and insulin.

Next, the AdipoR1/R2 expressions were analyzed in a mouse model in which insulin deficiency was induced by streptozotocin (STZ) treatment (Non-Patent Document 18). The STZ treatment enhanced the AdipoR1/R2 mRNA levels significantly in the liver, skeletal muscle, and adipose tissues of mice, which was shown to be restored by insulin administration. Moreover, the reduction of AdipoR1/R2 mRNAs in myocytes and hepatocytes was confirmed *in vitro.*

Analyses of the AdipoR1/R2 expressions in a mouse model of hyperinsulinemia caused by obesity-related insulin resistance (ob/ob mice) showed that expressions of AdipoR1/R2 were significantly reduced in the muscles and adipose tissues of ob/ob mice. The binding of adiponectin to membrane fractions of the above tissues was significantly decreased in ob/ob mice as compared to wild type mice, and AMP kinase activation by adiponectin was also decreased in the above tissues of ob/ob mice.

The above results reveal that insulin negatively regulates the expression levels of adiponectin receptors and adiponectin sensitivity, and that obesity-related insulin resistance/hyperinsulinemia causes downregulation of adiponectin receptors and adiponectin resistance.

Furthermore, the present inventors analyzed the mechanism behind insulin-mediated decrease of AdipoR1/R2 gene expression. Interestingly, the PI3-kinase inhibitor LY294002 negates the AdipoR1/R2 mRNA level reduction effect of insulin, whereas PD98059, a mitogen-activated protein kinase activation inhibitor, did not affect the above effect of insulin. Thus, the downregulation of adiponectin receptors by insulin was suggested to be mediated by the PI3-kinase signaling pathway.

Moreover, the present inventors examined the events downstream of PI3-kinase regarding the regulation of adiponectin receptor expression by insulin. It has been found that Akt, a downstream effector of PI3-kinase, phosphorylates transcriptional activators of the forkhead family, which results in inhibition of transcriptional activities of these phosphorylated transcriptional activators. On the other hand, AdipoR1/R2 genes comprise in their promoter an element capable of binding to the forkhead family transcriptional activator, FoxoI. Therefore, mouse FoxoI (wild type and constitutively active type) was transfected into the C2C12 cell line, and then the cell line was treated with insulin to observe AdipoR1/R2 expressions. The results showed that FoxoI enhanced AdipoR1/R2 expressions, and that insulin downregulates adiponectin receptor expression via inactivation of FoxoI.

In summary, the present inventors revealed, for the first time, that AdipoR1/R2 expressions in insulin target tissues are inversely correlated with plasma insulin levels, and that insulin negatively regulates the expression levels of adiponectin receptors through the PI3-kinase/FoxoI pathway. Not only AdipoR1/R2 agonists, but also a strategy to increase AdipoR1/R2 was shown to be effective for the first time, for the treatment of insulin resistance and type 2 diabetes. Thus, the present invention relates to therapeutic agents for diabetes mediating AdipoR1/R2 regulation, and more specifically provides the following:
[1] An adiponectin receptor expression-enhancing agent, comprising a FoxoI protein or a FoxoI gene.
[2] An adiponectin resistance-improving agent, comprising a FoxoI protein or a FoxoI gene as an active ingredient.
[3] An insulin resistance-improving agent, comprising a FoxoI protein or a FoxoI gene as an active ingredient.
[4] An obesity-preventing or improving agent, comprising a FoxoI protein or a FoxoI gene as an active ingredient.
[5] A diabetes-preventing or treating agent, comprising a FoxoI protein or a FoxoI gene as an active ingredient.
[6] An arteriosclerosis-preventing or treating agent, comprising a FoxoI protein or a FoxoI gene as an active ingredient.
[7] An adiponectin receptor expression-enhancing agent, comprising a PI3 (Phosphoinositide 3)-kinase-FoxoI pathway inhibitor.
[8] The adiponectin receptor expression-enhancing agent of [7], wherein the PI3-kinase-FoxoI pathway inhibitor is a PI3-kinase inhibitor.
[9] The adiponectin receptor expression-enhancing agent of [8], wherein the PI3-kinase inhibitor is LY294002.
[10] An adiponectin resistance-improving agent, comprising as an active ingredient the adiponectin receptor expression-enhancing agent of any one of [7] to [9].
[11] An insulin resistance-improving agent, comprising as an active ingredient the adiponectin receptor expression-enhancing agent of any one of [7] to [9].
[12] An obesity-preventing or improving agent, comprising as an active ingredient the adiponectin receptor expression-enhancing agent of any one of [7] to [9].
[13] A diabetes-preventing or treating agent, comprising as an active ingredient the adiponectin receptor expression-enhancing agent of any one of [7] to [9].
[14] An arteriosclerosis-preventing or treating agent, comprising as an active ingredient the adiponectin receptor expression-enhancing agent of any one of [7] to [9].
[15] An adiponectin receptor expression-suppressing agent, comprising insulin or insulin gene.
[16] A method of screening for an adiponectin receptor expression-enhancing agent, an adiponectin resistance-improving agent, an insulin resistance-improving agent, an obesity-improving agent, a therapeutic agent for diabetes, or a therapeutic agent for arteriosclerosis, comprising the step of contacting a test substance with PI3-kinase and a PI3-kinase substrate.
[17] A method of screening for an adiponectin receptor expression-enhancing agent, an adiponectin resistance-improving agent, an insulin resistance-improving agent, an obesity-improving agent, a therapeutic agent for diabetes, or a therapeutic agent for arteriosclerosis, wherein the method comprises the step of contacting a test substance with Akt and FoxoI.

The present invention revealed that insulin is involved in the regulation of adiponectin receptors and the mechanism of regulation, and at the same time, achieved specific agents for enhancing adiponectin receptor expression for the first time. Agents that enhance the expression of adiponectin receptors resolve both adiponectin resistance and insulin resistance, and thus, will be useful as novel agents for treating insulin resistance or type 2 diabetes.

### Brief Description of the Drawings

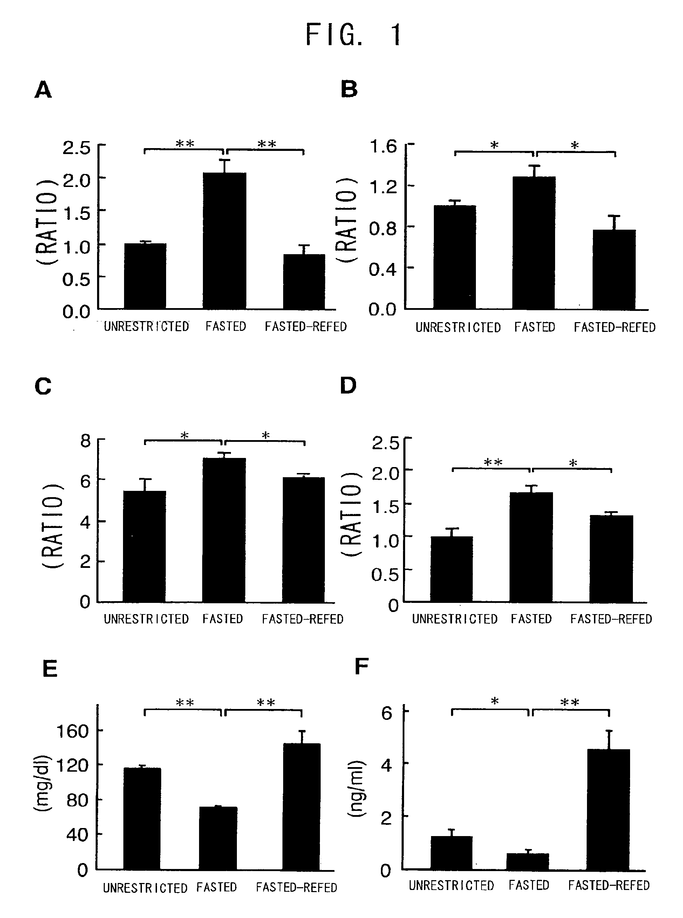

Fig. 1 shows the effects of nutrition control on the expression of AdipoR1/R2 genes. The expression levels of AdipoR1 (A and C) and AdipoR2 (B and D) in liver (A and B) and skeletal muscle (A and B), the blood glucose level (E), and the plasma insulin level (F) of mice were compared under conditions of unrestricted feeding, fasting, and refeeding. The unrestricted feeding group (ad lib) could feed freely (lane 1). The fasted group was separated from food for 48 hrs (lane 2). The refed group was allowed to freely feed for 6 hrs after 48 hrs of fasting (lane 3). Total RNA was prepared from the above tissues using TRIzol. A real-time PCR method was used to quantify AdipoR mRNAs. The primer sets and probes were as described in the Experimental Procedures. The total large amount of each AdipoR transcript was obtained by normalization to β-actin transcript amount. The results are shown as the ratio to the AdipoR1 value in the liver of the unrestricted feeding group. Each bar represents the mean ±standard error (S.E.)(n = 3) (*, p < 0.05; **, p < 0.01; compared with fasted mice).

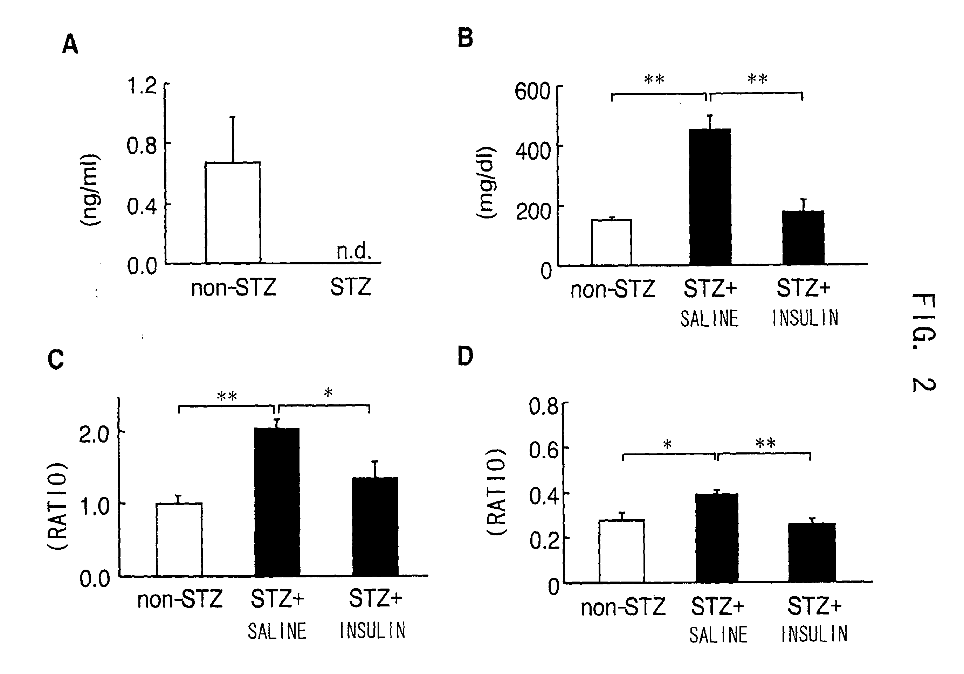

Fig. 2 shows the levels of plasma insulin (A), blood glucose (B), and mRNA of AdipoR1 (C), and AdipoR2 (D) in control mice and STZ-treated mice, with or without insulin. Total RNA was prepared and AdipoR mRNAs were quantified as described in the legend of Fig. 1. The results are shown as the ratio to the AdipoR1 value in the skeletal muscle of non-STZ mice. Each bar represents the mean ± S.E. (n = 3)(*, p < 0.05, **, p < 0.01, compared with STZ mice without insulin treatment).

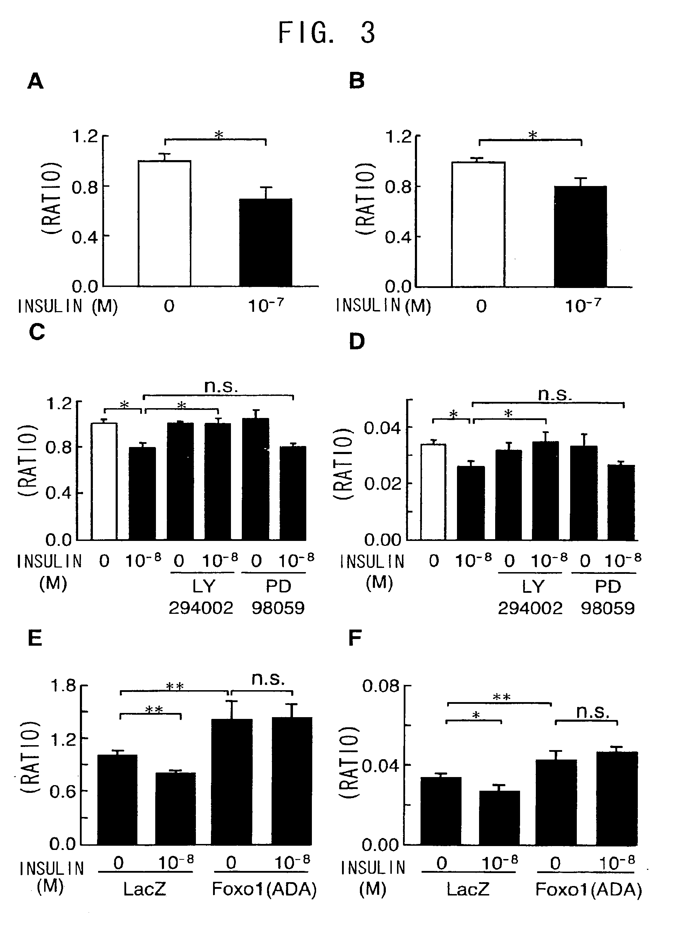

Fig. 3 shows the amount of mRNAs of AdipoR1 (A, C, and E) and AdipoR2 (B, D, and F) in mouse hepatocytes (A and B) or C2C12 myocytes (C-F), treated with or without insulin, incubated with or without LY294002 or PD98059 (C and D) or transfected with adenoviruses containing LacZ or FoxoI (E and F). Hepatocytes were isolated from mouse liver and incubated as described in the Experimental Procedures. The cells were then incubated for 6 hrs with or without the indicated concentrations of insulin. Total RNA was prepared and AdipoR mRNAs were quantified as described in the legend of Fig. 1. The results are shown as the ratio to the AdipoR1 value in a control solvent. Each bar represents the mean ± S.E. (n = 3)(*, p < 0.05, **, p < 0.01, compared with cells treated with solvent).

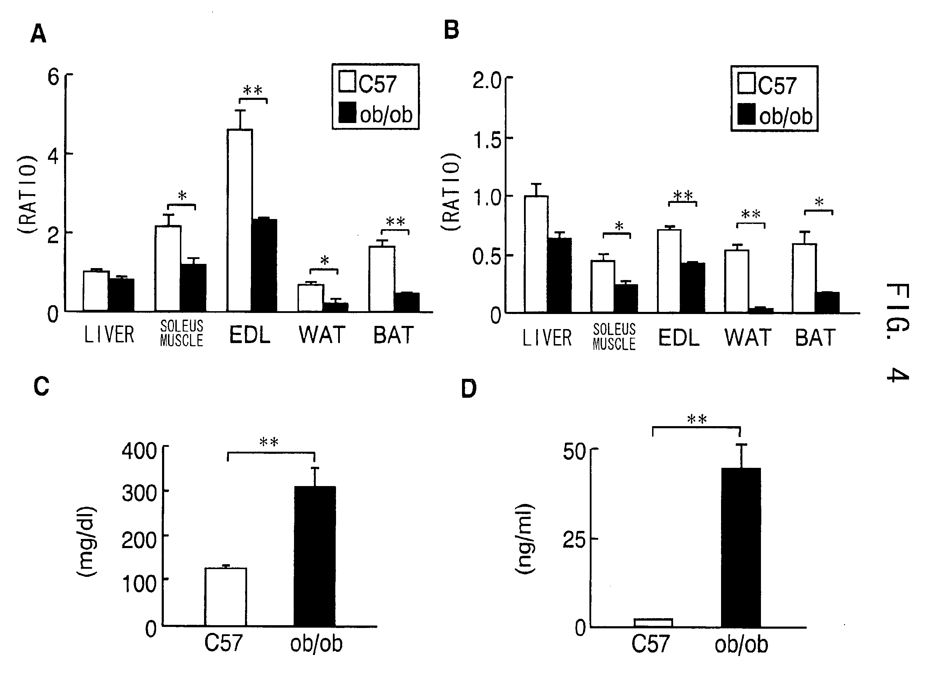

Fig. 4 shows the expression of AdipoR1 (A) and AdipoR2 (B) mRNAs in liver, soleus muscle, EDL, WAT, and BAT, and the levels of blood glucose (C) and plasma insulin (D) in wild-type (C57BL/6) and ob/ob mice. Preparation of total RNA and quantification of AdipoR mRNAs were performed as described in the legend of Fig. 1. The results are shown as the ratio to the AdipoR1 value in the liver of wild-type mice. Each bar represents the mean ± S.E. (n = 3)(*, p < 0.05, **, p < 0.01, between wild-type and ob/ob mice).

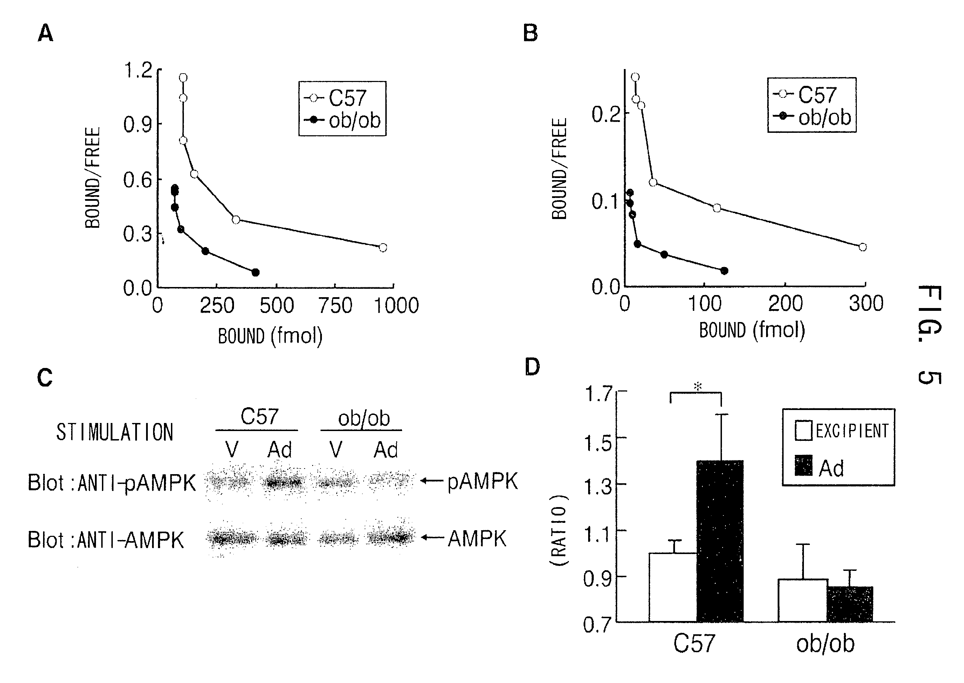

Fig. 5 shows the binding of globular (gAd) (A) or full-length adiponectin (Ad) (B) to the membrane fraction of skeletal muscles and AMPK activation by adiponectin in the skeletal muscle (C and D) of wild-type (C57BL/6) and ob/ob mice. Scatchard plot analysis of the binding of gAd or Ad to the membrane fraction of the skeletal muscle of wild type (C57BL/6) or ob/ob mice is shown (A and B). Phosphorylated α-subunit of AMPK in the skeletal muscle from wild type (C57BL/6) and ob/ob mice (C) was treated for 5 min with full-length adiponectin (Ad, 50 µg body weight) or with solvent. Immunoblotting analysis was performed using anti-pAMPK and anti-AMPK antibodies. The amount of pAMPK was normalized by the amount of AMPK from the same sample. The results are shown as the ratio to the value for the solvent-treated wild-type mice. Each bar represents the mean ± S.E. (n = 5-7)(*, p < 0.05, **, p< 0.01, compared with wild-type mice).

### Best Mode for Carrying Out the Invention

The present invention relates to adiponectin receptor expression-enhancing agents comprising FoxoI protein or FoxoI gene. The present inventors discovered that insulin regulates the expression of adiponectin receptors, and that the regulation is mediated by the PI3-kinase signaling pathway, and confirmed that forced expression of FoxoI (constitutively active type), a member of the PI3-kinase signaling pathway, reduces the inhibition of adiponectin receptor (AdipoR1/R2) expression by insulin. Using the diabetes mouse model, the present inventors also discovered for the first time that AdipoR1/R2 expression levels regulate the binding of adiponectin and the activation of AMPK by adiponectin, referred to as "adiponectin sensitivity". FoxoI is a forkhead transcription factor, also termed FKHR. The FoxoI protein and FoxoI gene of the present invention include both the wild type and the constitutively active type. The amino acid sequence of mouse FoxoI (wild type) protein is shown in SEQ ID NO: 1, and the cDNA sequence is shown in SEQ ID NO: 2. The amino acid sequence of the mouse FoxoI (constitutively active type) is shown in SEQ ID NO: 3. The amino acid sequence of human FoxoI protein is shown in SEQ ID NO: 4, and the cDNA sequence is shown in SEQ ID NO: 5.

The FoxoI protein and FoxoI gene can be prepared by methods commonly known to those skilled in the art. For example, mRNAs prepared from cells expressing human or mouse FoxoI protein, such as skeletal muscle cells, hepatocytes, and adipose tissues, are used as templates to prepare a cDNA library according to standard methods. Hybridization is carried out using a part or the entire nucleotide sequence of SEQ ID NO: 2 or 5, or a part or the entire polynucleotide encoding the amino acid sequence of SEQ ID NO: 3 as a probe to prepare FoxoI cDNA. Alternatively, the mRNAs prepared as above may be used as templates for RT-PCR with primers comprising a part of the nucleotide sequence of SEQ ID NO: 2 or 5, or a part of a polynucleotide encoding the amino acid sequence of SEQ ID NO: 3. Furthermore, the cDNA may be inserted into an appropriate expression vector, and introduced into host cells, expressed, and purified, to prepare a desired FoxoI protein.

The host and vector system may be selected depending on the purpose from an expression system such as a prokaryotic system, eukaryotic system, or cell free system, and a combination of vectors that match these expression systems. Examples of prokaryotic systems include *E. coli, Bacillus subtilis,* and *Actinomyces.* Yeasts, fungi, cultured animal cells, and such may be used as eukaryotic systems.

When *E. coli* is used as host, vectors such as pET, pPRO, and pBAD may be used. For efficient purification, vectors which express the desired protein as a fusion protein with other proteins may be selected. For example, vectors such as pGEX which express the desired protein as a fusion protein with GST (glutathione S-transferase), pCAL which expresses the desired protein as a fusion protein with CBP (calmodulin binding peptide), and pFLAG which expresses the desired protein as a fusion protein with an eight amino acid tag (Asp-Tyr-Lys-Asp-Asp-Asp-Asp-Lys) may be used. Examples of vectors for the *Bacillus subtilis* host include, pUB110, pAMα1, and such, and an example of a vector for the *Actinomyces* host is pIJ702. Vectors such as YES2 and pESC may be used for yeasts. When using insect cells and mammalian cells as host, baculovirus may be used. An example of a baculovirus vector in mammalian cells is pAcCAGMCS 1.

FoxoI proteins expressed in hosts can be purified by known techniques. FoxoI proteins expressed as a fusion protein with GST or His₆ may be purified by affinity chromatography on a glutathione sepharose column or nickel column, respectively. FoxoI proteins expressed using the above FLAG expression vector can be purified by affinity chromatography using commercially available monoclonal antibodies against the FLAG peptide.

The FoxoI protein of the present invention includes "proteins structurally similar and functionally equivalent to the FoxoI protein" (described below as "FoxoI-like proteins") because proteins that are structurally similar to the above FoxoI protein and have a function similar to FoxoI protein can be also utilized as adiponectin receptor expression-enhancing agents. "FoxoI-like proteins" include naturally-occurring mutants of human or mouse FoxoI, FoxoI homologues and orthologues isolated from other organisms (mammals such as rats, guinea pigs, rabbits, dogs, cats, horses, sheep, donkeys, monkeys, chimpanzees, and birds), and artificial mutants of human or mouse FoxoI. More specifically, they are "proteins comprising an amino acid sequence with a deletion, substitution, addition and/or insertion of one or more amino acids in the amino acid sequence of SEQ ID NO: 1, 3, or 4", and "proteins encoded by a polynucleotide that hybridizes with the nucleotide sequence of SEQ ID NO: 2 or 5 under stringent conditions".

Herein, the number of amino acids that are added, deleted, inserted, and/or substituted is not limited as long as the action to reduce the insulin-mediated AdipoR1 and/or AdipoR2 expression inhibition is retained. Usually, the number of such mutations is 10% of the total number of amino acids or less, preferably 3% of the total number of amino acids or less, and more preferably 1% of the total number of amino acids or less.

FoxoI-like proteins can be prepared by methods commonly known to those skilled in the art. For example, the DNA of a protein with high homology to FoxoI may be isolated from skeletal muscle cells, adipocytes, and hepatocytes of mammals including humans under stringent hybridization conditions, using a part of the nucleotide sequence of SEQ ID NO: 2 or 5 as a probe, after which the FoxoI-like protein can be prepared by expressing the DNA. Alternatively, FoxoI-like proteins may be prepared by expressing a polynucleotide encoding a FoxoI-like protein, as described later.

Those skilled in the art can appropriately select the above-mentioned stringent hybridization conditions. For example, pre-hybridization is carried out in a hybridization solution containing 25% formamide (50% formamide under more stringent conditions), 4x SSC, 50 mM Hepes (pH 7.0), 10x Denhardt's solution, and 20 µg/ml denatured salmon sperm DNA at 42°C overnight. A labeled probe is added and hybridization is carried out by incubation at 42°C overnight. Post-hybridization washes are carried out under different levels of stringency including moderately stringent "1x SSC, 0.1% SDS, 37°C", highly stringent "0.5x SSC, 0.1% SDS, 42°C", and more highly stringent "0.2x SSC, 0.1% SDS, 65°C" conditions. As the stringency level of post-hybridization washes increases, polynucleotides more highly homologous to the probe sequence are expected to be isolated. The above-described combinations of SSC, SDS, and temperature are merely examples of wash conditions. Those skilled in the art can achieve the same stringencies as those described above by appropriately combining the above factors or others (such as probe concentration, probe length, hybridization period, etc) that affect hybridization stringency.

A polypeptide encoded by the polynucleotide thus isolated using hybridization will usually comprise an amino acid sequence highly homologous to the polypeptides identified by the present inventors. "Highly homologous" refers to sequence homology of at least 40% or more, preferably 60% or more, further preferably 80% or more, further preferably 90% or more, further preferably at least 95% or more, further preferably at least 97% or more (for example, 98% to 99%). Amino acid sequence identity can be determined, for example, using the BLAST algorithm according to Karlin and Altschul (Proc. Natl. Acad. Sci. USA. 87:2264-2268, 1990; Proc. Natl. Acad. Sci. USA. 90: 5873-5877, 1993). A program designated BLASTX has been developed based on this algorithm (Altschul et al., J. Mol. Biol. 215: 403-410, 1990). For analysis of amino acid sequence identity, the BLASTX program uses, for example, a score of 50 and a word length of 3 as parameters. When using BLAST and Gapped BLAST programs, the default parameters of the respective program are used. Specific methodology for performing BLAST analyses is publicly available at http://www.ncbi.nlm.nih.gov.

Whether the polypeptides prepared as above act to reduce insulin-mediated AdipoR1 and/or AdipoR2 expression inhibition may be confirmed by methods known to those skilled in the art. To give an example, the polypeptides may be expressed in mouse myogenic cell lines such as the C2C12 cell line, using adenoviruses as described in Example 6, the cells are then treated with insulin, and subsequently, the expression levels of AdipoR1/R2 mRNAs in the cell line may be measured using primers comprising the nucleotide sequence of SEQ ID NO: 10, 11, 13, or 14, to confirm the action to enhance AdipoR1 and/or AdipoR2 expression. Adenovirus vectors may be constructed according to the method described in Non-Patent Document 22.

In addition, polynucleotides encoding a FoxoI-like protein can be used, like FoxoI, as an adiponectin receptor expression-enhancing agent. The FoxoI gene of the present invention includes "polynucleotides encoding FoxoI-like proteins". Polynucleotides encoding FoxoI-like proteins of the present invention may be prepared by introducing site-specific or random mutations to the nucleotide sequence of SEQ ID NO: 2 or 5, using genetic engineering methods commonly known to those skilled in the art, such as mutation introduction by PCR and cassette mutagenesis. Alternatively, the nucleotide sequence of SEQ ID NO: 2 or 5 introduced with a mutation(s) may be synthesized using a commercial DNA synthesizer.

The above FoxoI gene of the present invention can be used in gene therapy in humans and the like, by expressing FoxoI proteins and enhancing adiponectin receptor expression. Although the FoxoI gene may be used as is in the form of a polynucleotide as an adiponectin receptor expression-enhancing agent, it is preferable to make an adiponectin receptor expression-enhancing agent by inserting the FoxoI gene into an appropriate vector. The vector may be selected from vectors used in gene therapy, including for example retrovirus vectors, adenovirus vectors, adeno-associated virus (AAV) vectors, adenovirus/AAV hybrid vectors, artificial liposome vectors, etc.

In addition, the present invention relates to adiponectin receptor expression-enhancing agents comprising a PI3-kinase/FoxoI pathway inhibitor. The present inventors revealed for the first time that the regulation of adiponectin receptor expression by insulin is mediated by the PI3-kinase pathway. Examples of PI3-kinase-FoxoI pathway inhibitors include the PI3-kinase inhibitor LY294002 (CAS No.: [154447-36-6], 2-(4-morpholinyl)-8-phenyl-4H-1-benzopyran-4-one) and wortmannin (CAS No.: [19545-26-7]). A preferable example is LY294002, but the inhibitors are not limited thereto. As long as a substance inhibits PI3-kinase activity, it is included in the adiponectin receptor expression-enhancing agents of the present invention. Moreover, other PI3-kinase-FoxoI pathway inhibitors such as Akt activity inhibitors and FoxoI phosphorylation inhibitors are also included in the present invention's adiponectin receptor expression-enhancing agents.

The present inventors showed for the first time that decreased adiponectin receptor expression leads to adiponectin resistance, and that insulin resistance is evoked as a result of impaired adiponectin actions. Therefore, the present invention's adiponectin receptor expression-enhancing agents can be used as agents for improving adiponectin resistance and insulin resistance. In addition to suppressing obesity, diabetes, and arteriosclerosis by improving insulin resistance, adiponectin is also known to suppress arteriosclerosis through direct actions on arterial walls. Thus, the adiponectin receptor expression-enhancing agents of the present invention could be very useful as obesity-preventing or improving agents, diabetes-preventing or treating agents, or arteriosclerosis-preventing or treating agents.

When using a FoxoI protein, a FoxoI gene, or a PI3-kinase-Akt pathway inhibitor of the present invention as a pharmaceutical agent, the substance can either be directly administered to a patient, or administered as a formulation prepared by a commonly known pharmaceutical method. For example, the substance may be appropriately combined with a pharmaceutically acceptable carrier or vehicle, more specifically, sterilized water, saline, plant oil, emulsifying agent, suspension, detergent, stabilizing agent, or such for formulation, and then administered. Administration to patients may be carried out by known methods such as intraarterial administration, intravenous administration, subcutaneous administration, intramuscular administration, oral administration, and transdermal administration. The dosage varies depending on the age and body weight of patients and administration method, but those skilled in the art can appropriately select a suitable dose.

Furthermore, the present invention relates to adiponectin receptor expression-suppressing agents comprising insulin or insulin gene. As described above, insulin was shown to downregulate adiponectin receptor expression and thus evokes adiponectin resistance and insulin resistance. The adiponectin receptor expression-suppressing agents comprising insulin or insulin gene are important as reagents for further elucidating the mechanism of adiponectin resistance and insulin resistance. The amino acid sequence of mouse insulin is shown in SEQ ID NO: 6 and the cDNA sequence of mouse insulin is shown in SEQ ID NO: 7. The amino acid sequence of human insulin is shown in SEQ ID NO: 8 and the cDNA sequence of human insulin is shown in SEQ ID NO: 9.

Insulin and insulin gene can be prepared by methods commonly known to those skilled in the art. For example, insulin gene may be obtained by preparing mRNA from mouse or human pancreatic cells, and performing RT-PCR using a part of the nucleotide sequence of SEQ ID NO: 7 or 9 as primer. Alternatively, a cDNA library prepared from mouse or human pancreatic cells may be screened using a part of the nucleotide sequence of SEQ ID NO: 7 or 9 as a probe to prepare the insulin gene. An insulin gene prepared as above may be expressed using an appropriate host-vector system for purification to prepare a desired insulin protein.

The above insulin protein is thought to be able to reduce the amount of adiponectin receptors expressed in cells by contacting it with cells that express adiponectin receptors. Similarly, an insulin gene introduced into adiponectin receptor-expressing cells can produce cells with reduced adiponectin receptor expression amount. Such cells may be used for elucidating the mechanisms of adiponectin resistance, insulin resistance, and diabetes.

Furthermore, the insulin gene may be introduced into animals such as mice, to produce animals with sustainably reduced adiponectin receptor expression levels. The insulin gene may be introduced into animals by introducing a fragment of the insulin gene by the particle gun method and such, but preferably, it is introduced using the aforementioned vectors for gene therapy. The above animals are thought to be useful as animal models for adiponectin resistance, insulin resistance, and diabetes, and especially for elucidating the mechanisms and for developing therapeutic agents and therapeutic methods.

Furthermore, the present invention relates to methods of screening for adiponectin receptor expression-enhancing agents, adiponectin resistance-improving agents, insulin resistance-improving agents, obesity-improving agents, or for therapeutic agents for diabetes or arteriosclerosis, in which the methods of screening comprise the step of contacting test substances with PI3 kinase and PI3 kinase substrates. As described in the Examples, a PI3-kinase inhibitor was confirmed to inhibit the suppression of adiponectin receptor expression by insulin, clarifying that the inhibition of adiponectin receptor expression by insulin is mediated through the PI3-kinase pathway. Therefore, the activity to inhibit PI3-kinase can be used as a marker for selecting adiponectin receptor expression-enhancing agents.

The first embodiment of the present invention comprises an *in vitro* system using purified PI3-kinase. The above system comprises the steps of contacting a test substance with PI3-kinase and a PI3-kinase substrate, detecting the PI3-kinase activity in the presence of the test substance, comparing the activity of PI3-kinase with or without the test substance, and selecting a test substance when PI3-kinase activity in the presence of the test substance is lower than that in the absence of the test substance. PI3-kinase catalyzes the reaction that produces PI(3,4,5)P₃ using PI(4,5)P₂ as a substrate. The above reaction activity can be measured by known methods. For example, phosphorylation of phosphatidylinositol substrates by PI3-kinase may be carried out with P³² or P³³ in the presence or absence of a test substance. Radioactive phosphorylation products are extracted, and then separated and detected by thin-layer chromatography. By comparing the activities of PI3-kinase with or without the test substance, the test substance's inhibition of PI3-kinase activity can be measured. In addition, PI3-kinase may be reacted with a substrate in the presence of a test substance, and the thus produced PI(3,4,5)P₃ may be measured by ELISA. This method can be conducted easily using a commercial PI3-kinase ELISA kit (Echelon Biosciences, Inc). In addition, a FRET (fluorescence resonance energy transfer)-based PI3-kinase assay (PI Profiler^{™}, Upstate) may be used for the measurement.

The second embodiment of the present invention includes a system which comprises the step of contacting a test substance with PI3-kinase and a PI3-kinase substrate in cells, by incubating PI3-kinase-expressing cells with the test substance. In this system, following the step of contacting the test substance with PI3-kinase in the cells, the following steps can be carried out: measuring PI(3,4,5)P₃ in the cells; comparing the PI(3,4,5)P₃ measurements in the absence or presence of the test substance; and selecting the substance when the measured value in the presence of the test substance is lower than that in the absence of test substance. PI(3,4,5)P₃ may be measured by preparing cell lysates, and PI(3,4,5)P₃ in the cell lysates may be measured by an immunoassay using a specific antibody.

Examples of test substances according to the present invention include nucleic acids, proteins, polypeptides, synthetic low molecular weight compounds, cell extracts, and extracts of natural products, but are not restricted thereto, and may be other substances. Test substances obtained by a screening method of the present invention are expected to enhance adiponectin receptor expression through PI3-kinase inhibition, and thus may be used as adiponectin receptor expression-enhancing agents, adiponectin resistance-improving agents, insulin resistance-improving agents, obesity-improving agents, or therapeutic agents for diabetes or arteriosclerosis.

Moreover, the present invention relates to methods of screening for adiponectin receptor expression-enhancing agents, adiponectin resistance-improving agents, insulin resistance-improving agents, obesity-improving agents, or therapeutic agents for diabetes or arteriosclerosis, in which the screening methods comprise the step of contacting Akt with FoxoI. In the methods of the present invention, phosphorylation of FoxoI is used as a marker for screening the above substances. The Examples of the present invention show that insulin inhibits AdipoR1/R2 expressions via inactivation of FoxoI. Accordingly, phosphorylation of FoxoI at specific residues suppresses the activity of FoxoI to transcribe target genes. On the other hand, the promoter region of AdipoR1/R2 contains a cis-acting element that is capable of binding to FoxoI. These suggest that FoxoI is phosphorylated by Akt and inactivated, which in turn suppresses adiponectin receptor expression. Thus, phosphorylated FoxoI measurement can be used as a marker when screening for adiponectin receptor expression-enhancing agent.

As one embodiment of the present invention, a method of the present invention may be performed as an *in vitro* assay system, using purified Akt and FoxoI. The above system comprises the steps of contacting a test substance with Akt and FoxoI, measuring the phosphorylated FoxoI in the presence or absence of the test substance, comparing the measurements of phosphorylated FoxoI with or without the test substance, and selecting the test substance when the measured value of phosphorylated FoxoI in the presence of the test substance is less than the activity in the absence of the test substance. The above method can be performed using known methods. For example, Akt is contacted with FoxoI in the presence of a test substance *in vitro,* and then reacted with phospho-specific antibodies to measure the amount of phosphorylated FoxoI by an immunoassay. The phospho-specific antibody preferably detects residues phosphorylated by Akt. Examples of such an antibody include the commercially available Phospho-FKHR(Ser256) Antibody, and Phospho-FKHR(Thr24)/FKHR1(Thr32) Antibody (both from Cell Signaling Technology).

Another embodiment of the methods of the present invention includes a system comprising the step of contacting a test substance with Akt and FoxoI in cells, by incubating Akt and FoxoI expressing cells in the presence or absence of the test substance. In this system, following the above step, the following steps are performed: measuring the phosphorylated FoxoI in the cells; comparing the measurements of phosphorylated FoxoI in the presence or absence of the test substance; and selecting the test substance when the measured value of phosphorylated FoxoI in the presence of the substance is less than the activity in the absence of the test substance. The phosphorylated FoxoI in the cells may be measured, for example, by preparing cell lysates, and then measuring by a method similar to that described above.

Examples of test substances for the methods of the present invention include nucleic acids, proteins, polypeptides, synthetic low molecular weight compounds, cell extracts, and extracts of natural products, but are not restricted thereto, and may be other substances. Substances obtained by the screening methods of the present invention are expected to have an action to suppress the inactivation of FoxoI by insulin, and thereby enhance adiponectin receptor expression. Thus, such substances may be used as adiponectin receptor expression-enhancing agents, adiponectin resistance-improving agents, insulin resistance-improving agents, obesity-improving agents, or therapeutic agents for diabetes or arteriosclerosis.

All prior art references cited herein are incorporated into this description by references.

### Examples

The present invention will be explained more specifically below based on Examples; however, the present invention is not limited thereto. All the reagents used in the Examples were obtained from the sources described in references (Non-Patent Documents 6, 10, 14, and 19).

### [Example 1] Fasting-induced expression increase and refeeding-induced expression decrease of AdipoR1/R2 genes

To elucidate whether or not the expression of AdipoR1 and/or AdipoR2 is regulated under physiological and/or pathophysiological conditions, the expression levels of AdipoR1 and AdipoR2 in insulin and adiponectin target organs were examined in mice under conditions of unrestricted feeding, fasting, and refeeding.

Fifteen-week male C57BL/6 mice obtained from Charles River Breeding Laboratories (Washington, MA) were kept in colony cages, maintaining a 12-hr light/dark cycle. The mice were provided or deprived of food for 48 hrs from the start of the dark cycle (9:00 p.m.). The refed group was provided with food after 48 hrs of fasting, and then sacrificed 6 hrs after refeeding for tissue isolation. The mice were given a high fat diet containing 1152 g of fat (Benibana (safflower oil, high oleic acid type), Japan, containing 46% oleic acid (18:ln-9) and 45% linolenic acid (18:2n-6) in total fatty acids), 1191.6 g of casein (No. 19, Oriental Yeast, Tokyo, Japan), 633.6 g of sucrose (No. 13, Oriental Yeast, Tokyo), 50.4 g of a vitamin mix (No. 20 (AIN76), Oriental Yeast), 352.8 g of a mineral mix (No. 20 (AIN76), Oriental Yeast), 201.6 g of cellulose powder (No. 19, Oriental Yeast), 18 g of DL-methionine (Wako Pure Chemical Industries, Osaka, Japan), and 360 ml of water; 3600 g in total(Non-Patent Document 19).

Real time PCR was performed to quantify Adipo receptor mRNAs (Non-Patent Document 14). Total RNA was prepared from cells or tissues using TRIzol (GIBCO/BRL) according to the instructions provided by the manufacturer. The relative amount of each AdipoR transcript was obtained by normalization to the amount of actin transcript in the same cDNA (Non-Patent Document 14). The primer sets and probes used for the quantification are shown below.

| mAdipoR1 | | |
|---|---|---|
| Forward primer: | acgttggagagtcatcccgtat | (SEQ ID NO: 10) |
| Reverse primer: | ctctgtgtggatgcggaagat | (SEQ ID NO: 11) |
| Probe: | cctgctacatggccacagaccacct | (SEQ ID NO: 12) |

| mAdipoR2 | | |
|---|---|---|
| Forward primer: | tcccaggaagatgaagggtttat | (SEQ ID NO: 13) |
| Reverse primer: | ttccattcgttcgatagcatga | (SEQ ID NO: 14) |
| Probe: | atgtccccgctcctacaggccc | (SEQ ID NO: 15) |

The expression levels of AdipoR1 and AdipoR2 mRNAs in the liver dramatically increased after 48 hrs of fasting, and were rapidly restored by refeeding to the same level as in natural feeding conditions (Figs. 1A and 1B). A similar AdipoR2 mRNA expression regulation due to fasting and refeeding was also observed in skeletal muscles (Figs. 1C and 1D).

### [Example 2] An inverse correlation between the plasma insulin level and the expression level of AdipoR1/R2 gene

The above results in Example 1 confirm that the expression of AdipoR1/R2 gene is regulated in response to fasting and feeding, suggesting the involvement of a particular hormone(s) or nutrient(s) in the above regulation. Because insulin is a classic nutritional hormone, plasma insulin levels of the above mice under unrestricted-feeding, fasting, and refeeding states were measured using an insulin immunoassay (Shibayagi, Gunma, Japan).

As expected, fasting decreased the plasma insulin level (Fig. IF), whereas refeeding increased the plasma insulin level in parallel with the blood glucose level (Fig. 1E). These findings suggest that the plasma insulin values inversely correlate with the expressed amount of AdipoR1/R2 mRNAs, presenting the possibility that insulin may control AdipoR1/R2 expression.

### [Example 3] STZ treatment-induced expression increase and insulin-induced expression decrease of AdipoR1/R2

To further examine the effect of insulin on AdipoR1/R2 expression, mice were treated with streptozotocin (STZ) and AdipoR1/R2 expression was observed. STZ disrupts pancreatic βcells, and causes acute insulin deficiency. To induce diabetes, mice were intraperitoneally administered twice with 0.2-0.3 ml of a 50 mM sodium citrate solution (pH 4.5) containing STZ (200 mg/kg body weight). Control mice were injected with a 50 mM sodium citrate solution (pH 4.5). For three days after injection, plasma glucose levels were measured, and diabetes was confirmed (glucose value > 250 mg/dl). Human regular insulin (1 unit/kg, Eli Lilly, Indianapolis, IN) dissolved in 0.2 ml of saline was administered to the STZ + insulin treated group. The mice in the STZ + saline treated group and non-STZ treated group were intraperitoneally administered with 0.2 ml of saline. After insulin or saline administration, the animals were fasted for 6 hrs and then sacrificed to collect the hind limb skeletal muscles.

STZ treatment made plasma insulin disappear (Fig. 2A), and at the same time, caused a significant increase of plasma glucose (Fig. 2B, lanes 1 and 2). The STZ and insulin treated group exhibited a lower plasma glucose value as compared to the group treated with STZ only (Fig. 2B, lanes 2 and 3).

Next, the amount of AdipoR1 and AdipoR2 mRNA in skeletal muscle and liver extracts was measured as described above. In STZ-treated mice, the AdipoR1 and AdipoR2 mRNA in the skeletal muscle respectively showed a 103% and 38% increase (Figs. 2C and 2D), which was almost completely restored by insulin treatment. These findings further support the possibility that insulin reduces AdipoR1/R2 mRNA levels. STZ treatment had little effect on the AdipoR1/R2 mRNA levels in the liver, whereas insulin significantly reduced the AdipoR1/R2 mRNA levels in the liver of STZ-treated mice (data not shown). These data may be explained by the findings that inflammation may decrease AdipoR1/R2 mRNA levels (Tsuchida, Yamauchi, and Kadowaki, manuscript in preparation) and that STZ treatment can induce inflammation in the liver (Non-Patent Document 23).

### [Example 4] Decrease in in vitro AdipoR1/R2 mRNA levels due to insulin.

The direct effect of insulin on AdipoR1/R2 expression in Hepatocytes was examined. Hepatocytes were prepared from fasted 8-week-old male C57BL/6 mice by collagenase perfusion method (Non-Patent Document 20). Aliquots of 0.8 x 10⁶ cells were plated into Williams medium E supplemented with 5% (v/v) fetal calf serum, 10 nM dexamethasone, 1 nM insulin, 100 units/ml penicillin, and 100 µg/ml streptomycin, on a collagen I-coated 12-well dish. After incubation at 37°C, 5% CO₂ for 5 hrs, the cells were washed twice with PBS, and then incubated for 6 hrs in Williams medium E supplemented with 1% bovine serum albumin. The cells were then transferred to Williams medium E supplemented with 1% bovine serum albumin and 100 nM insulin, and incubated with or without 100 nM insulin for 6 hrs. The hepatocytes were harvested to measure the total amount of AdipoR1/R2 mRNA. Insulin reduced the total amount of AdipoR1/R2 mRNA (Figs. 3A and 3B).

To further explore the possibility that insulin may reduce AdipoR1/R2 mRNA levels, the effect of insulin on the expression levels of AdipoR1/R2 mRNA in C2C12 myocytes was observed *in vitro.* Induction of myogenic differentiation was carried out according to a previously reported method (Non-Patent Document 10). Briefly, mouse C2C12 myoblasts were cultured in Dulbecco's modified Eagle's medium- high glucose-(DMEMH; 90%) with 10% (v/v) FBS. When the cells reached 80% confluence, the medium was replaced with a low serum differentiation medium (98% DMEMH, 2.5% (v/v) horse serum). The medium was changed every day to induce the differentiation of myoblasts into myotubes. By day 5, the cells had differentiated into multinucleated contractile myotubes. Treatment with 10 nM insulin indeed reduced the expression levels of AdipoR1/R2 mRNA in the above C2C12 myocytes after 6 hrs (Figs. 3C and 3D).

### [Example 5] Suppression of insulin-induced reduction in AdipoR1/R2 mRNA levels by a phosphoinositide 3-kinase (PI3-kinase) inhibitor

C2C12 cells were incubated in DMEMH supplemented with 1% bovine serum albumin for 12 hrs, and then exposed to insulin transduction pathway inhibitors (10 µM LY294002 or 10 µM PD98059) 30 min before the addition of insulin. The cells were harvested 6 hrs after the addition of insulin to measure the AdipoR1/R2 mRNA levels in the C2C12 myocytes treated with insulin for 6 hrs (Figs. 3C and 3D). The PI3-kinase inhibitor LY294002 canceled the AdipoR1/R2 mRNA level reduction effect of insulin. In contrast, PD98059, an inhibitor of mitogen-activated protein kinase activation, did not essentially affect the response to insulin. The above results led to the conclusion that the induction of AdipoR1/R2 gene expression may be mediated by the PI3-kinase branched pathway of the insulin signal transduction pathway. The inhibitory effects shown in Figs. 3C and 3D exhibited maximum value against all of the reagents tested. Further, the points were verified by dose-response experiments (data not shown).

Many, if not all, of the known actions of insulin are ascribable to the tyrosine phosphorylation in which the insulin receptor of IRS-1 and IRS-2 plays a part, and this phosphorylation triggers kinase cascades involving the PI3-kinase/Akt pathway and the MAP kinase pathway (Non-Patent Documents 26-28). The effect of insulin on AdipoR1/R2 gene expression was hardly affected by the MEK inhibitor PD98059, and thus does not appear to require activation of the mitogen-activated protein kinase cascade. In contrast, the results with LY294002 clearly implicated the PI3-kinase signal transduction pathway. The results with the PI3-kinase inhibitor leads to the conclusion that the induction of AdipoR1/R2 gene expression may be mediated by the PI3-kinase branched pathway of the insulin signal transduction pathway.

### [Example 6] Enhancement of AdipoR1/R2 mRNA levels by FoxoI and inhibition of insulin-induced reduction of AdipoR1/R2 mRNA levels

The PI3-kinase signal transduction pathway is thought to be involved in the insulin-mediated regulation of several genes, particularly, the regulation of the gene for insulin-like growth factor binding protein (IGF-BP1) (Non-Patent Document 29). Recently, Akt, an effector downstream of PI3-kinase, was shown to phosphorylate several transcriptional activators of the forkhead family including FoxoI *in vitro* and in intact cells. When specific residues of these activators are phosphorylated, the phosphorylated activators become isolated in the cytoplasm, and as a result, their ability to activate the transcription of target genes is inhibited (Non-Patent Documents 30-32). Because IGF-BP1 gene contains in its promoter a *cis*-acting element capable of binding to FoxoI, the insulin dependent suppression of IGF-BP1 gene could, at least partially, be due to Akt-mediated phosphorylation and inactivation of the forkhead transcriptional activators (Non-Patent Document 29).

The AdipoR1/R2 genes also contain in their promoters a *cis*-acting element capable of binding to FoxoI. Thus, the insulin dependent suppression of AdipoR1/R2 genes could, at least partially, be due to PI3-kinase/Akt-mediated phosphorylation and inactivation of the forkhead transactivators (Non-Patent Documents 21 and 22). Thus, the effect of FoxoI expression on insulin dependent suppression of AdipoR1/R2 genes was observed. C2C12 cells were transfected with adenoviruses containing wild-type mouse FoxoI cDNA and constitutively active form of FoxoI (T24A/S253D/S316A (ADA)) cDNA (Non-Patent Documents 21 and 22) according to a previously described method (Non-Patent Document 10) with minor modifications. Differentiation was induced as described above. At day 5, the above cells were treated with the indicated concentrations of insulin, and then analyzed.

The expression of constitutively active FoxoI (FoxoI-ADA) induced a dramatic increase in AdipoR1/R2 mRNA levels. Insulin failed to restore the above effect of FoxoI (Figs. 3E and 3F). These findings suggest that FoxoI enhances AdipoR1/R2 expression levels and that insulin suppresses AdipoR1/R2 mRNA expressions through inactivation of FoxoI.

### [Example 7] Downregulation of AdipoR1/R2 expression levels in ob/ob mice

The expression of AdipoR1 and R2 in insulin-sensitive tissues was examined using insulin-resistant ob/ob mice and C57BL/6 mice as a control. The ob/ob mice are genetically obese mice that exhibit obesity, fatty liver, and hyperinsulinemia, due to a deficiency in the leptin gene. Insulin-sensitive tissues such as liver, skeletal muscles (soleus muscle and extensor digitorum longus (EDL)), and adipose tissues (white adipose tissue (WAT) and brown adipose tissue (BAT)) were examined. The results are shown in Figs. 4A and 4B. In the control C57BL/6 mice, AdipoR1 was expressed most abundantly in skeletal muscles, whereas AdipoR2 was expressed most in the liver. Interestingly, AdipoR1 and AdipoR2 were both expressed more abundantly in EDL than in soleus muscles. Both AdipoR1 and R2 were expressed in both WAT and BAT adipose tissues.

The expressions of both AdipoR1 and R2 in ob/ob mice were significantly less than in C57BL/6 mice in all of the examined tissues except the liver (Figs. 4A and 4B). The expression level of AdipoR1 in the liver, soleus muscle, EDL, WAT, and BAT of the ob/ob mice was 79.4%, 54.5%, 50.5%, 31.9%, and 28.1% of the level in the wild-type mice, respectively (Fig. 4A). The expression level of AdipoR2 in the liver, soleus muscle, EDL, WAT, and BAT of the ob/ob mice was 63.3%, 51.4%, 59.5%, 7.9%, and 29.4% of that in the wild-type mice, respectively (Fig. 4B).

### [Example 8] The correlation between reduction in AdipoR1/R2 expression levels and "adiponectin sensitivity" in ob/ob mice

The association between AdipoR1/R2 expression levels and the effects of adiponectin in each tissue was examined. Adiponectin contributes to the enhancement of insulin-sensitivity via activation of AMPK and PPARα. Thus, the activation of AMPK by adiponectin and the amount of adiponectin bound to the membrane fraction of skeletal muscles were measured in obesity-related insulin resistance model mice (ob/ob mice) and wild-type mice (C57BL/6 mice).

The amount of adiponectin bound to the membrane fraction of skeletal muscles was determined as follows. First, recombinant mouse full-length adiponectin (Ad) was prepared as described in references (Non-Patent Documents 10 and 14). Tyrosine of synthetic adiponectin was labeled with ¹²⁵I using IODO-beads (Pierce) in the presence of Na ¹²⁵I (2,000 Ci/mmol, Amersham Pharmacia Biotech) according to the manufacturer's protocol. The membrane fraction of skeletal muscles was purified by sucrose gradient centrifugation, and incubated at 4°C for 1 hr in a binding buffer (ice-cold phosphate-buffered saline/10% skim milk) containing an indicated concentration of [¹²⁵I]-adiponectin (2,000 cpm/ng protein) and non-labeled competitors. Two hours later, when binding experiments were conducted at 4°C, establishment of binding equilibrium was confirmed. The cells were washed three times with ice-cold phosphate-buffered saline, and the membrane-bound radioactivity was determined using a y-counter (Non-Patent Documents 10 and 14). Non-specific binding was determined by over 200-fold unlabeled adiponectin. Specific binding was calculated by subtracting the nonspecific binding from the total binding. The values shown in the results are the average of three determinations from three experiments.

The activation of AMPK by adiponectin was measured as follows. Fifteen-week-old male ob/ob mice or age-matched C57BL/6 mice were anesthetized with pentobarbital, and then injected with the above recombinant adiponectin (50 µg/10 g of body weight) through the inferior vena cava. Skeletal muscles were collected five minutes after adiponectin injection. The amount of phosphorylated AMPK in skeletal muscles was determined by western blotting (Non-Patent Documents 10 and 14).

Interestingly, the binding of adiponectin was more robust in the membrane fraction of skeletal muscles of wild-type mice than that of ob/ob mice (Figs. 5A and 5B). Scatchard plot analysis shows that both the high affinity binding site and low affinity binding site for globular adiponectin and full-length adiponectin were decreased in the skeletal muscles of ob/ob mice compared with those in wild-type mice, suggesting that the numbers of both AdipoR1 and AdipoR2 were decreased (Figs. 5A and 5B) (Non-Patent Document 14). Moreover, adiponectin could activate AMPK in the skeletal muscles of wild-type mice, but could not do so in the skeletal muscles of ob/ob mice (Figs. 5c and 5D). These findings suggest that AdipoR1/R2 expression levels regulate adiponectin binding and activation of AMPK by adiponectin, referred to as "adiponectin sensitivity".

Thus, the present inventors discovered that AdipoR1/R2 expressions are downregulated in the obesity-related insulin-resistance model ob/ob mice. Previously, the inventors showed that plasma adiponectin level was reduced in ob/ob mice (Non-Patent Document 12), and that such changes in plasma adiponectin level play a causal role in the regulation of insulin sensitivity. Taken together, these findings raise an interesting possibility that changes in AdipoR1/R2 expression levels, in addition to the plasma adiponectin level, may play a causal role in the regulation of insulin sensitivity. This possibility is consistent with the previous report by the inventors that the AdipoR1 or AdipoR2 expression reduction by their corresponding siRNA is associated with the insulin-sensitizing effect of adiponectin (Non-Patent Document 14). The present inventors proved for the first time that AdipoR1 or AdipoR2 expression reduction in model mice is associated with the insulin-sensitizing effect of adiponectin *in vivo.*

It is expected that chronic overeating and its consequent increase in insulin level will lead to decreased AdipoR1/R2 expressions. Such a decrease will cause both adiponectin resistance and insulin resistance. The decrease in AdipoR1/R2 mRNAs leads to a decrease in adiponectin binding, which in turn leads to a decrease in the adiponectin effects, referred to as "adiponectin resistance", completing a so-called "vicious cycle". These findings may provide a novel molecular mechanism against long-term insulin elevation that leads to insulin resistance, comprising downregulation of adiponectin receptors in addition to the well known downregulation of insulin receptors.

To conclude, AdipoR1/R2 expressions in insulin target tissues appear to be inversely correlated with the plasma insulin level. The present invention demonstrated for the first time that insulin negatively regulates the expression level of adiponectin receptors through the PI3-kinase/FoxoI pathway. These data suggest that not only AdipoR1/R2 agonists but also a strategy to increase AdipoR1/R2 can be a logical approach to provide a novel treatment concept for insulin resistance and type 2 diabetes.

### Industrial Applicability

Adiponectin receptor expression-enhancing agents of the present invention are capable of resolving both adiponectin resistance and insulin resistance, and thus will be useful as adiponectin resistance-improving agents, insulin resistance-improving agents, obesity-improving agents, or therapeutic agents for diabetes or arteriosclerosis. The screening methods of the present invention makes use of the fact that insulin regulates adiponectin receptor expression via the PI3-kinase pathway, and will be particularly useful for discovering candidate substances for adiponectin resistance-improving agents, insulin resistance-improving agents, obesity-improving agents, or therapeutic agents for diabetes or arteriosclerosis.

## Claims

1. An adiponectin receptor expression-enhancing agent, comprising a FoxoI protein or a FoxoI gene.

2. An adiponectin resistance-improving agent, comprising a FoxoI protein or a FoxoI gene as an active ingredient.

3. An insulin resistance-improving agent, comprising a FoxoI protein or a FoxoI gene as an active ingredient.

4. An obesity-preventing or improving agent, comprising a FoxoI protein or a FoxoI gene as an active ingredient.

5. A diabetes-preventing or treating agent, comprising a FoxoI protein or a FoxoI gene as an active ingredient.

6. An arteriosclerosis-preventing or treating agent, comprising a FoxoI protein or a FoxoI gene as an active ingredient.

7. An adiponectin receptor expression-enhancing agent, comprising a PI3 (Phosphoinositide 3)-kinase-FoxoI pathway inhibitor.

8. The adiponectin receptor expression-enhancing agent of claim 7, wherein the PI3-kinase-FoxoI pathway inhibitor is a PI3-kinase inhibitor.

9. The adiponectin receptor expression-enhancing agent of claim 8, wherein the PI3-kinase inhibitor is LY294002.

10. An adiponectin resistance-improving agent, comprising as an active ingredient the adiponectin receptor expression-enhancing agent of any one of claims 7 to 9.

11. An insulin resistance-improving agent, comprising as an active ingredient the adiponectin receptor expression-enhancing agent of any one of claims 7 to 9.

12. An obesity-preventing or improving agent, comprising as an active ingredient the adiponectin receptor expression-enhancing agent of any one of claims 7 to 9.

13. A diabetes-preventing or treating agent, comprising as an active ingredient the adiponectin receptor expression-enhancing agent of any one of claims 7 to 9.

14. An arteriosclerosis-preventing or treating agent, comprising as an active ingredient the adiponectin receptor expression-enhancing agent of any one of claims 7 to 9.

15. An adiponectin receptor expression-suppressing agent, comprising insulin or insulin gene.

16. A method of screening for an adiponectin receptor expression-enhancing agent, an adiponectin resistance-improving agent, an insulin resistance-improving agent, an obesity-improving agent, a therapeutic agent for diabetes, or a therapeutic agent for arteriosclerosis, comprising the step of contacting a test substance with PI3-kinase and a PI3-kinase substrate.

17. A method of screening for an adiponectin receptor expression-enhancing agent, an adiponectin resistance-improving agent, an insulin resistance-improving agent, an obesity-improving agent, a therapeutic agent for diabetes, or a therapeutic agent for arteriosclerosis, wherein the method comprises the step of contacting a test substance with Akt and FoxoI.
